# EUROPEAN PATENT SPECIFICATION

(11) **EP 3 925 526 B1**
(45) Date of publication and mention of the grant of the patent: **12.08.2026**
(21) Application number: 21177188.6
(22) Date of filing: 01.06.2021
(51) Int. Cl.: A61B 5/02, A61B 5/029, A61B 5/00

(54) **HEMODYNAMIC PARAMETER ANALYSIS APPARATUS AND HEMODYNAMIC PARAMETER ANALYSIS PROGRAM**
VORRICHTUNG ZUR ANALYSE VON HÄMODYNAMISCHEN PARAMETERN UND PROGRAMM ZUR ANALYSE VON HÄMODYNAMISCHEN PARAMETERN
APPAREIL ET PROGRAMME D'ANALYSE DE PARAMÈTRE HÉMODYNAMIQUE

(30) Priority: 15.06.2020 JP 2020103191
(43) Date of publication of application: 22.12.2021
(73) Proprietor: Nihon Kohden Corporation, Shinjuku-ku Tokyo (JP)
(72) Inventor: SATO, Haruka, Saitama (JP); KOBAYASHI, Naoki, Saitama (JP)
(74) Representative: Grünecker Patent- und Rechtsanwälte PartG mbB

(56) References cited:
- EP-A1- 2 314 211
- WO-A1-2019/188688
- WO-A1-2020/059641
- US-A1- 2008 139 958

## Description

### TECHNICAL FIELD

The presently disclosed subject matter relates to a hemodynamic parameter analysis apparatus and a hemodynamic parameter analysis program.

### BACKGROUND ART

Monitoring hemodynamic parameters of a patient in a medical site such as a hospital is indispensable for managing a state of the patient during and after surgery.

A medical worker monitors, for example, arterial pressure, venous pressure, urine volume, and the like during or after surgery, and checks that necessary blood is supplied from a heart to each part of a body of the patient.

A function of the heart can be evaluated, for example, by measuring an amount of blood output from the heart for one minute (cardiac output (CO)). In general, the cardiac output is invasively measured by detecting a change in a temperature of blood flowing through a pulmonary artery using a Swan-Ganz catheter. In recent years, a technique for estimating the cardiac output based on an electrocardiogram and a photoplethysmogram has been developed in consideration of a burden on the patient (for example, see JP2005-312947A).

As a result of measuring the cardiac output, if the cardiac output is low, some problems may occur in blood circulation. However, if only the cardiac output is measured, it is not possible to distinguish whether a decrease in the cardiac output is caused by a decrease in a contractile function of the heart or a decrease in an amount of blood returning to the heart, that is, an amount of circulating blood. Since treatment for the patient is different depending on whether the contractile function of the heart is lowered or the amount of circulating blood is decreased, it is necessary to separate these two cases.

In relation to this, a charted classification of severity of heart failure of a patient has been known in the related art. In this classification, presence or absence of pulmonary congestion and peripheral circulatory failure of the patient is classified based on pulmonary capillary wedge pressure (PCWP) invasively measured by the Swan-Ganz catheter and a cardiac index (CI) calculated based on the cardiac output.
EP 2314211 A1 teaches a biological parameter displaying apparatus configured to display first coordinates in which a first biological parameter of a patient, which is measured by a first measuring unit, is set as an X-axis, and a second biological parameter, which is measured by the second measuring unit, is set as the Y-axis.

US2008/0139958 provides a method for estimating cardiac output and pulmonary artery wedge pressure based on an impedance signal detected by an implantable defibrillator.

An evaluation support system for supporting the evaluation of the state of a circulatory system based on at least two types of measurement data is known from WO 2020/059641 A1.

WO 2019/188688 teaches a management system configured to acquire measurement data related to symptoms of heart failure.

The pulmonary capillary wedge pressure is applied to a distal end of a catheter when the pulmonary artery is blocked by a balloon of the catheter and a blood flow is closed at a periphery of the pulmonary artery. Since the pulmonary capillary wedge pressure reflects pulmonary capillaries and left atrial pressure downstream of the pulmonary artery, the pulmonary capillary wedge pressure is an indicator of left atrial preload and the pulmonary congestion. The medical worker can formulate an appropriate treatment policy for the patient with reference to a classification result of the above-described classification.

However, in order to use the above-described classification, it is necessary to invasively measure the pulmonary capillary wedge pressure, and thus a burden on the patient for the measurement may be large.

### SUMMARY

The presently disclosed subject matter has been made to solve the above-described problem. Therefore, a main object of the presently disclosed subject matter is to provide a hemodynamic parameter analysis apparatus and a hemodynamic parameter analysis program that are capable of analyzing hemodynamic parameters of a patient while reducing a burden on the patient for measurement.

The above-described object of the presently disclosed subject matter is achieved by the following.

According to a first aspect of the presently disclosed subject matter, a hemodynamic parameter analysis apparatus according to claim 1 is provided.

A second aspect of the presently disclosed subject matter provides a hemodynamic parameter analysis program according to claim 21.

According to the presently disclosed subject matter, the hemodynamic parameters of the patient are analyzed based on central venous pressure (CVP) and the cardiac output that are calculated based on physiological information of the patient. Therefore, the hemodynamic parameters of the patient can be estimated while reducing the burden on the patient for measurement.

### BRIEF DESCRIPTION OF DRAWINGS

FIG. 1 is a block diagram illustrating a schematic hardware configuration of a physiological information display system according to an embodiment; FIG. 2 is a block diagram illustrating a schematic hardware configuration of a control unit illustrated in FIG. 1;
FIG. 3 is a functional block diagram illustrating main functions of the control unit illustrated in FIG. 1;
FIG. 4 is a flowchart illustrating a processing procedure of a hemodynamic parameter analysis method of the control unit;
FIG. 5 is a schematic diagram illustrating an example in which an analysis result of hemodynamic parameters is displayed in a matrix form;
FIG. 6 is a schematic diagram illustrating an example in which the analysis result of hemodynamic parameters is displayed in a table form;
FIG. 7 is a schematic diagram illustrating a region where congestion and peripheral circulatory failure may occur on a coordinate plane with central venous pressure and cardiac output as coordinate axes;
FIG. 8 is a schematic diagram illustrating an example in which the analysis result of hemodynamic parameters is displayed on a coordinate plane;
FIG. 9 is a schematic diagram illustrating a case in which threshold values of the central venous pressure and the cardiac output are adjusted;
FIG. 10 is a schematic diagram illustrating a case in which a plurality of threshold values are set for the central venous pressure and the cardiac output;
FIG. 11 is a schematic diagram illustrating a basic monitor screen of a physiological information display apparatus;
FIG. 12 is a schematic diagram illustrating a history of the analysis result of hemodynamic parameters;
FIG. 13 is a schematic diagram illustrating a history of the analysis result of hemodynamic parameters;
FIG. 14 is a schematic diagram illustrating a case in which the analysis result of hemodynamic parameters and a measurement value and the like related to the analysis result are displayed together;
FIG. 15 is a schematic diagram illustrating a case in which the analysis result of hemodynamic parameters and measurement values and the like related to the analysis result are displayed together;
FIG. 16 is a schematic diagram illustrating a case in which the analysis result of hemodynamic parameters and treatment information related to the analysis result are displayed together;
FIG. 17 is a flowchart illustrating a processing procedure for calculating venous pressure; and
FIG. 18 is a flowchart illustrating a processing procedure for calculating cardiac output.

### DESCRIPTION OF EMBODIMENTS

Hereinafter, embodiments of the presently disclosed subject matter will be described with reference to the accompanying drawings. In the drawings, the same members are denoted by the same reference numerals. Dimensional ratios in the drawings are exaggerated for convenience of description, and may be different from actual ratios.

### <Physiological Information Display System 100>

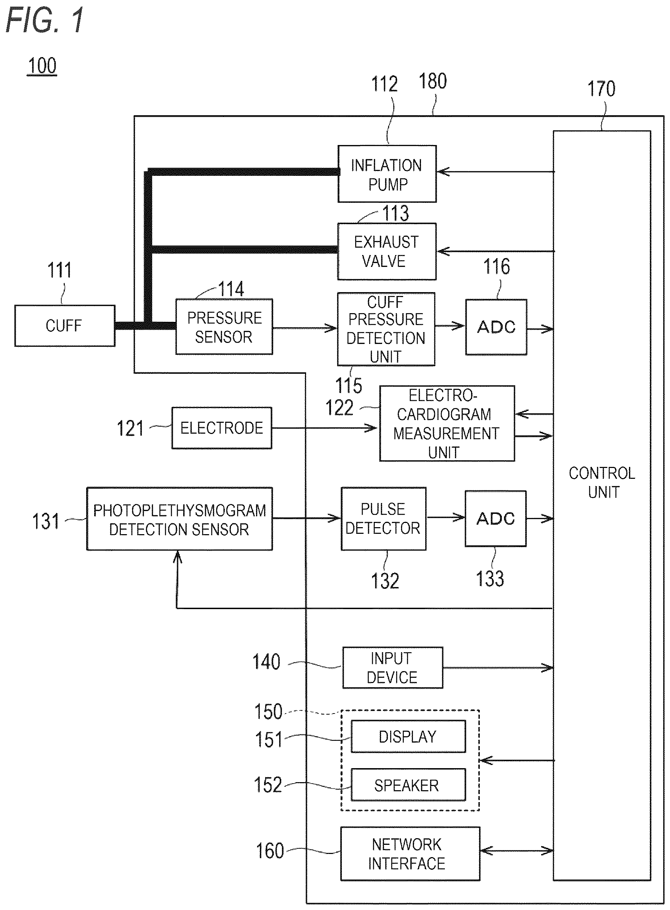

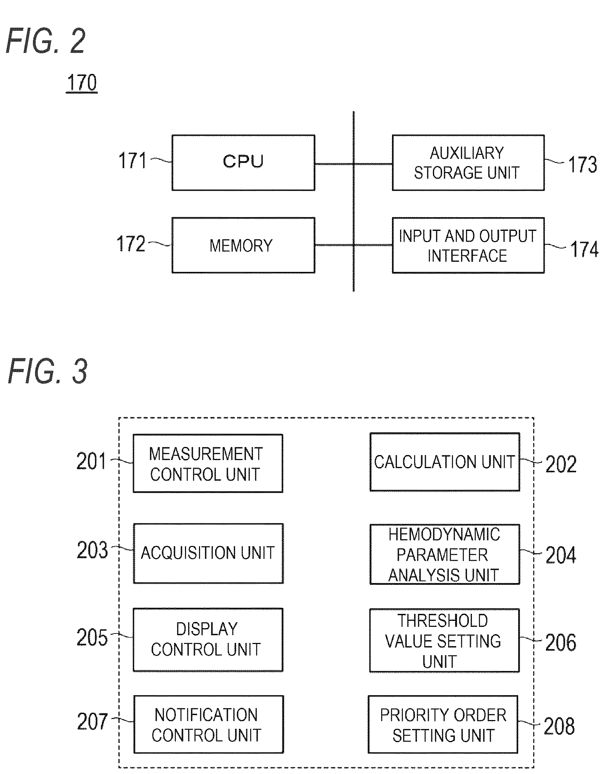

FIG. 1 is a block diagram illustrating a schematic hardware configuration of a physiological information display system 100 according to an embodiment, and FIG. 2 is a block diagram illustrating a schematic hardware configuration of a control unit 170 illustrated in FIG. 1.

The physiological information display system 100 (hereinafter, simply referred to as a "display system 100") according to the present embodiment is configured to non-invasively measure central venous pressure and cardiac output based on physiological information (for example, a pulse wave and an electrocardiogram) collected from a patient (a subject), and to analyze hemodynamic parameters of the patient or to display information on the hemodynamic parameters based on the measured central venous pressure and cardiac output. Therefore, a user (for example, a medical worker such as a doctor or a nurse) of the display system 100 can perform appropriate treatment on the patient according to an analysis result displayed on a display or the information on the hemodynamic parameters.

In the following, a case is described as an example in which the pulse wave and the electrocardiogram are acquired as the physiological information and the central venous pressure and the cardiac output are estimated based on the pulse wave and the electrocardiogram. However, the physiological information to be acquired is not limited to the pulse wave and the electrocardiogram, and other physiological information may be acquired instead of or in addition to the pulse wave and the electrocardiogram.

The central venous pressure is an index that reflects pressure (right atrial pressure) measured in a right atrium, and can reflect an amount of blood returning to the heart and right atrial preload. Therefore, since the central venous pressure reflects an amount of circulating blood, the medical worker can evaluate body congestion based on an increase of the central venous pressure. The cardiac output is an index that reflects a contractile function of the heart, and a decrease in the cardiac output indicates a possibility of a decrease in the contractile function of the heart.

However, in the related art, the central venous pressure and the cardiac output are generally measured invasively using, for example, a Swan-Ganz catheter. On the other hand, in the present embodiment, the physiological information is acquired and the central venous pressure and the cardiac output are estimated based on the physiological information, it is possible to prevent the central venous pressure and the cardiac output from being invasively measured for the patient, and to non-invasively measure the central venous pressure and the cardiac output. Accordingly, the burden on the patient for the measurement can be significantly reduced.

In the above-described classification, presence or absence of pulmonary congestion and peripheral circulatory failure is determined based on the invasively measured pulmonary capillary wedge pressure and cardiac output (cardiac index). However, in the present embodiment, instead of the invasively measured pulmonary capillary wedge pressure and cardiac output, hemodynamic parameters of the patient are analyzed using the non-invasively measured central venous pressure and cardiac output. Although the central venous pressure is different from the pulmonary capillary wedge pressure, it is considered that the pulmonary capillary wedge pressure can be substituted with the central venous pressure based on a viewpoint of evaluating congestion. This is because it is considered that, when the cardiac function is lowered and the heart cannot sufficiently output blood, a blood flow on a left side of the heart is congested and a right side of the heart is affected. Congestion is a main cause of exacerbation of heart failure. In this case, it is known that the central venous pressure increases.

As illustrated in FIG. 1, the display system 100 can include a cuff 111, an electrocardiogram measurement electrode 121, a photoplethysmogram detection sensor 131, and a physiological information display apparatus 180 (a hemodynamic parameter analysis apparatus). The cuff 111 is connectable to the physiological information display apparatus 180. The physiological information display apparatus 180 can include an inflation pump 112, an exhaust valve 113, a pressure sensor 114, a cuff pressure detection unit 115, an AD converter (ADC) 116, an electrocardiogram measurement unit 122, a pulse detector 132, an ADC 133, an input device 140, an output device (output unit) 150, a network interface 160, and a control unit 170.

The physiological information display apparatus (hereinafter, also simply referred to as a "display apparatus") 180 may be a medical apparatus (for example, a patient monitor) configured to analyze the hemodynamic parameters of the patient and display an analysis result. Hereinafter, a main configuration of the display system 100 will be described.

The cuff 111, the pressure sensor 114, the cuff pressure detection unit 115, and the ADC 116 function as a blood pressure measurement unit, and are configured to measure arterial blood pressure (systolic blood pressure and/or diastolic blood pressure) of the patient.

The cuff 111 is attached by winding an air bag around an upper arm of the patient. The inflation pump 112 is configured to send air into the air bag of the cuff 111 in response to an instruction from the control unit 170, and to increase pressure (hereinafter, referred to as "cuff internal pressure") in the air bag. Accordingly, pressure (hereinafter, referred to as "cuff pressure") on the upper arm of the patient by the cuff 111 can be increased. The exhaust valve 113 is configured to gradually exhaust the air in the air bag from the cuff 111 and to reduce the cuff internal pressure by opening the air to the atmosphere. Accordingly, the cuff pressure can be reduced.

The pressure sensor 114 is configured to detect the cuff internal pressure. A pulse wave (hereinafter referred to as a "cuff pulse wave") of the patient in a process of increasing and decreasing the cuff pressure is superimposed on the cuff internal pressure. The cuff pressure detection unit 115 is configured to extract the cuff pulse wave superimposed on the detected cuff internal pressure from the cuff internal pressure, and to output the cuff internal pressure and the extracted cuff pulse wave to the ADC 116 as an analog signal. The ADC 116 is configured to convert the analog signal of the cuff internal pressure and the cuff pulse wave into a digital signal and to transmit the digital signal to the control unit 170. The pressure sensor 114, the cuff pressure detection unit 115, and the ADC 116 may be integrated with the cuff 111. In this case, the control unit 170 receives the digital signal of the cuff pulse wave. Same or similarly, the pressure sensor 114 and the cuff pressure detection unit 115 may be integrated with the cuff 111, and the ADC 116 may be provided in the physiological information display apparatus 180.

The electrocardiogram measurement unit 122 is configured to continuously detect an electrocardiogram that indicates an action potential generated by excitement of myocardium of the patient, and to transmit the detected electrocardiogram data to the control unit 170. More specifically, the electrocardiogram measurement unit 122 is configured to detect an electrocardiogram via a plurality of the electrocardiogram measurement electrodes 121 attached to a predetermined portion of the body of the patient. The electrocardiogram data is stored in an auxiliary storage unit 173 (described later) of the control unit 170. The electrocardiogram has a plurality of heartbeat waveforms that are continuously generated on a time axis. The heartbeat waveform indicates a heartbeat, that is, a pulsation of the heart.

The photoplethysmogram detection sensor 131 (hereinafter, referred to as a "pulse sensor 131"), the pulse detector 132, and the ADC 133 function as a photoplethysmogram measurement unit, and are configured to detect a pulse wave of the patient using a photoplethysmogram method and to transmit data of the detected pulse wave to the control unit 170. The pulse detector 132 and the ADC 133 may be provided in the photoplethysmogram detection sensor 131, and the photoplethysmogram detection sensor 131 may transmit the pulse wave data to the control unit 170. Same or similarly, only the ADC 133 may be provided in the physiological information display apparatus 180.

The pulse sensor 131 is a SpO2 probe configured to measure arterial oxygen saturation (SpO2), and is attached to a peripheral portion (for example, a tip of a finger) of the body of the patient. In the present embodiment, the pulse sensor 131 is preferably attached to a tip of a left finger of the patient. However, the presently disclosed subject matter is not limited thereto. A photoplethysmogram measurement apparatus 130 can be used not only for a purpose of measuring the SpO2, but also for measuring pulse wave propagation time to be described later.

The pulse sensor 131 can include a light emission unit and a light reception unit, and is configured to irradiate the patient with red light or infrared light at a predetermined light emission timing and to receive transmitted light. The light emission unit can include, for example, a light emission diode configured to emit light with a wavelength of approximately 660 nm (red) or approximately 940 nm (infrared), and is configured to emit light toward a physiological surface (a fingertip) of the patient. The light reception unit can include, for example, a photodiode, and is configured to receive transmitted light transmitted through a blood vessel and a physiological tissue and to convert the transmitted light into an electrical signal corresponding to the transmitted light.

The pulse detector 132 is configured to detect a pulse wave from the electrical signal generated by the pulse sensor 131, and to output the pulse wave as a photoplethysmogram signal to the ADC 133. The ADC 133 is configured to convert the photoplethysmogram signal into a digital signal and to output the digital signal to the control unit 170.

The input device 140 is configured to accept input operation of a user who operates the display apparatus 180 and to generate an input signal corresponding to the input operation. The input device 140 can include, for example, a touch panel overlaid on a display 151 of an output device 150 to be described later, an operation button attached to a housing of the display apparatus 180, a mouse, a keyboard, or the like. The input signal generated by the input device 140 is transmitted to the control unit 170, and the control unit 170 is configured to execute predetermined processing according to the input signal.

The output device 150 is configured to output an analysis result of the hemodynamic parameters. The output device 150 can include a display 151 and a speaker 152. The display 151 functions as a display, and can be a liquid crystal display, an organic EL display, or the like attached to the housing of the display apparatus 180. The display 151 may be a display apparatus such as a transmissive or non-transmissive head-mounted display worn on a head of a user.

The speaker 152 is attached to the housing of the display apparatus 180, and is configured to issue an alarm to the user by voice. The analysis result of the hemodynamic parameters may be output by voice. The output device 150 can include a light emission unit that can include an LED or the like, and can be configured to issue an alert by light such as the LED.

The output device 150 is not limited to the display 151 and the speaker 152, and can include, for example, a printer configured to print and output the analysis result of the hemodynamic parameters.

The network interface 160 is configured to connect the control unit 170 to a communication network. Specifically, the network interface 160 can include processing circuits for various interfaces configured to communicate with an external device such as a server via a communication network, and is configured to conform to a communication standard for communication via the communication network. The communication network is a local area network (LAN), a wide area network (WAN), the Internet, or the like.

The control unit 170 is configured to analyze the hemodynamic parameters of the patient. The control unit 170 may be software and hardware that are configured to govern a main control of the physiological information display apparatus 180, or may be an independent device. For example, the control unit 170 may be a dedicated medical device configured to analyze the hemodynamic parameters, or may be a personal computer, a smartphone, a tablet terminal, or the like in which a hemodynamic parameter analysis program (hereinafter referred to as an "analysis program") for analyzing the hemodynamic parameters is installed. Further, the control unit 170 may be a wearable device or the like worn on the body (for example, an arm, a head, or the like) of the user.

As illustrated in FIG. 2, the control unit 170 can include a central processing unit (CPU) 171, a memory 172, an auxiliary storage unit 173, and an input and output interface 174.

The memory 172 which can be any non-transitory tangible storage medium, can include a read only memory (ROM) and a random access memory (RAM). The ROM stores various programs, parameters, and the like. The RAM can include a work area in which various programs and the like executed by the CPU 171 are stored. The CPU 171 is configured to load a program specified from various programs stored in the ROM or the auxiliary storage unit 173 on the RAM and to execute various types of processing in cooperation with the RAM.

The auxiliary storage unit 173 which can be any non-transitory tangible storage medium, can include, for example, a storage device (storage) such as a hard disk drive (HDD), a solid state drive (SSD), or a USB flash memory. The auxiliary storage unit 173 is configured to store an analysis program and various types of data. The auxiliary storage unit 173 is configured to store electrocardiogram data and pulse wave data as physiological information.

The input and output interface 174 functions as an interface between the CPU 171 and the input device 140 and the output device 150. The input and output interface 174 can include various communication modules configured to communicate with an input device such as a mouse and a keyboard, a drive module configured to drive the display 151 and the speaker, and the like.

The CPU 171 executes the analysis program, so that the control unit 170 controls each unit of the display system 100 to implement various functions. FIG. 3 is a functional block diagram illustrating main functions of the control unit 170. The control unit 170 functions as a measurement control unit (a physiological information acquisition unit) 201, a calculation unit 202, an acquisition unit 203, a hemodynamic parameter analysis unit 204, a display control unit 205, a threshold value setting unit 206, a notification control unit 207, and a priority order setting unit 208.

The measurement control unit 201 is configured to integrally control measurement apparatuses which are a blood pressure measurement unit, the electrocardiogram measurement unit 122, and the photoplethysmogram measurement unit, and to acquire data of the physiological information (the cuff pulse wave, the electrocardiogram, and the photoplethysmogram) from these measurement devices. The measurement control unit 201 is configured to manage a timing of analyzing the hemodynamic parameters of the patient, and to perform a necessary control for each measurement apparatus according to this timing. The hemodynamic parameters can usually be analyzed at a predetermined time interval. The predetermined time interval is not particularly limited by the medical worker when a condition of the patient is stable. However, the predetermined time interval may be set to, for example, 30 minutes to several hours. However, when a measurement request from the medical worker or the condition of the patient rapidly changes, the measurement control unit 201 can also perform control so as to analyze the hemodynamic parameters of the patient at a shorter time interval according to a degree of change in the condition of the patient.

The measurement control unit 201 is configured to, when the timing of analyzing the hemodynamic parameters of the patient is reached, output an instruction to start measurement to the blood pressure measurement unit and control the inflation pump 112 and the exhaust valve 113 according to the measured arterial blood pressure. The measurement control unit 201 is configured to output an instruction to start measurement to the electrocardiogram measurement unit 122 and the photoplethysmogram measurement unit at predetermined timings. The acquisition of the electrocardiogram and the photoplethysmogram has less burden on the subject as compared with the inflation by the cuff. Therefore, it is more preferable that the electrocardiogram and the photoplethysmogram are continuously measured and the cuff pulse wave is measured using the cuff at a predetermined timing.

The calculation unit 202 is configured to calculate the central venous pressure (hereinafter, also simply referred to as "venous pressure") of the patient and the cardiac output based on the physiological information of the patient acquired by the measurement control unit 201. In the present embodiment, since the venous pressure and the cardiac output of the patient can be measured by calculation, invasive measurement can be avoided. A specific method for calculating the venous pressure and the cardiac output will be described later.

### <Hemodynamic Parameter Analysis Method>

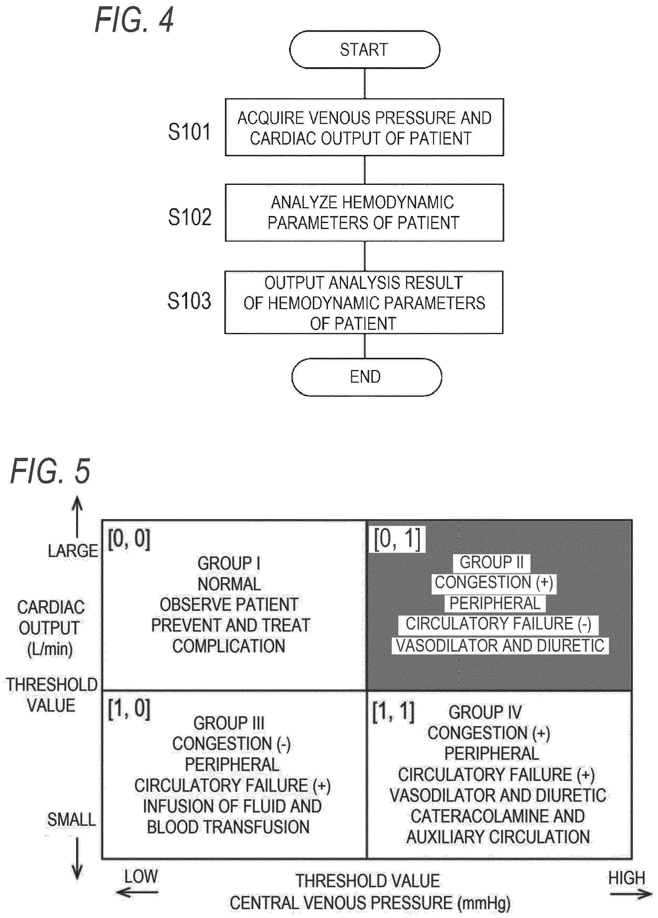

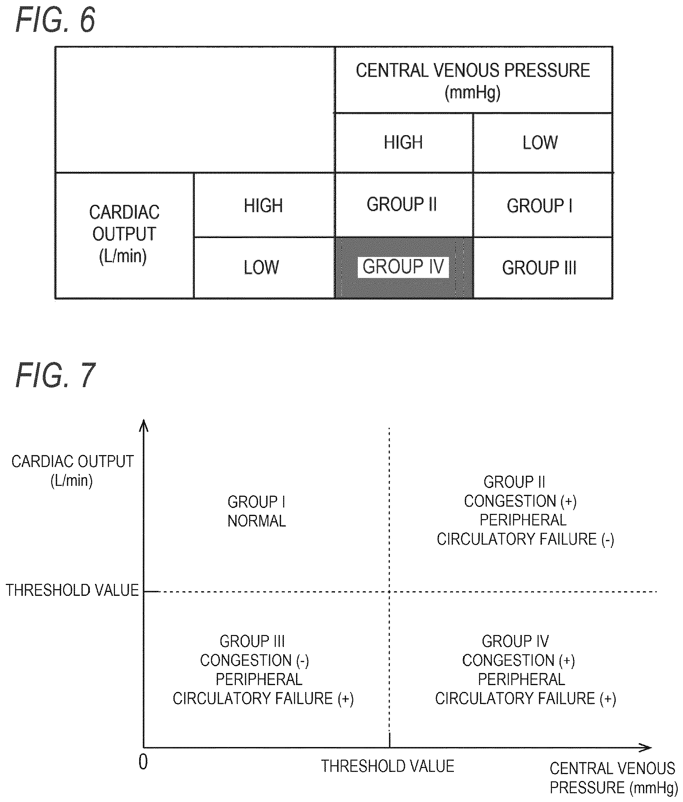

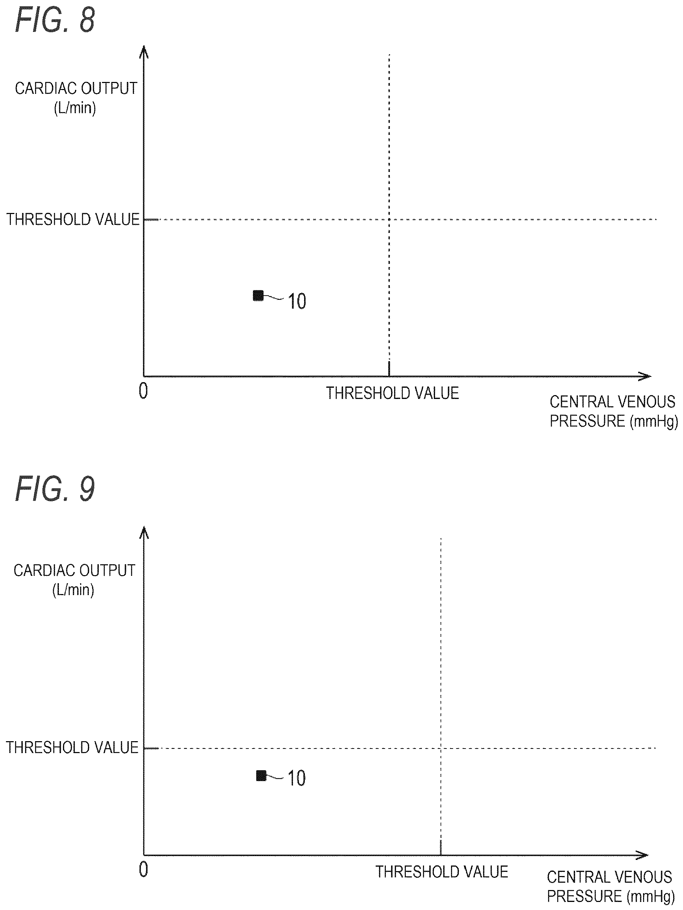

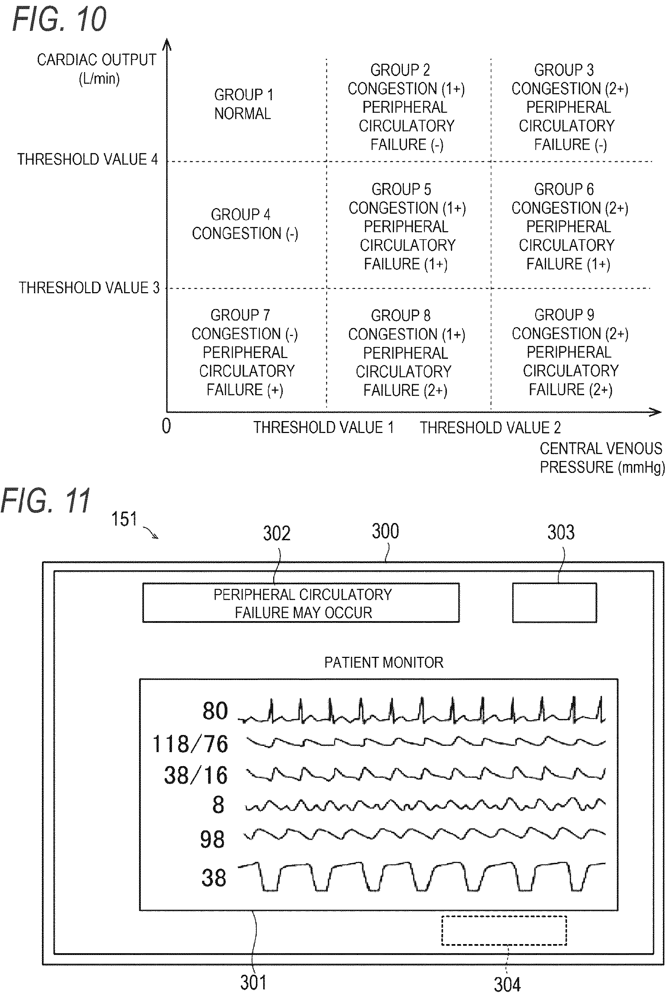

FIG. 4 is a flowchart illustrating a processing procedure of a hemodynamic parameter analysis method of the control unit 170. The processing of the flowchart in the drawing is implemented by the CPU 171 executing the analysis program. FIG. 5 is a schematic diagram illustrating an example in which an analysis result of the hemodynamic parameters is displayed in a matrix form, and FIG. 6 is a schematic diagram illustrating an example in which the analysis result of the hemodynamic parameters is displayed in a table form. FIG. 7 is a schematic diagram illustrating a region where congestion and peripheral circulatory failure may occur on a coordinate plane with the central venous pressure and the cardiac output as coordinate axes, and FIG. 8 is a schematic diagram illustrating an example in which the analysis result of the hemodynamic parameters is displayed on a coordinate plane. FIG. 9 is a schematic diagram illustrating a case in which threshold values of the central venous pressure and the cardiac output are adjusted, and FIG. 10 is a schematic diagram illustrating a case in which a plurality of threshold values are set for the central venous pressure and the cardiac output.

As illustrated in FIG. 4, first, the venous pressure and the cardiac output of the patient are acquired (step S101). The acquisition unit 203 acquires the venous pressure and the cardiac output that are calculated by the calculation unit 202 based on the physiological information of the patient. The venous pressure and the cardiac output are not limited to calculation results calculated by the calculation unit 202, but may be non-invasively measured by another system and may be acquired from a server (a computer) provided on the communication network via the network interface 160.

Next, the hemodynamic parameters of the patient are analyzed (step S102). The hemodynamic parameter analysis unit 204 analyzes the hemodynamic parameters of the patient based on the venous pressure and the cardiac output that are acquired in step 101. For example, based on the venous pressure and the cardiac output, the hemodynamic parameter analysis unit 204 analyzes at least one of the congestion and a blood output ability (a circulatory failure) of the heart of the patient.

More specifically, the hemodynamic parameter analysis unit 204 estimates a degree of the congestion in a blood vessel of the patient based on the acquired venous pressure, and estimates a degree of the circulatory failure of the patient based on the acquired cardiac output. Therefore, the medical worker can distinguish between two cases, that is, a case in which a contractile function of the heart of the patient is lowered and a case in which an amount of circulating blood is decreased. Further, the hemodynamic parameter analysis unit 204 determines a classification (correspondence relation between the degree of the congestion and the degree of the circulatory failure) corresponding to the degree of the congestion of the patient and the degree of the circulatory failure of the patient based on a classification group (a classification table) related to the condition of the patient. The classification group is generally classified in advance according to the degree of the congestion and the degree of the circulatory failure of human being. As will be described later, the classification group is classified into a plurality of groups (for example, groups I to IV) based on the threshold value of the venous pressure and the threshold value of the cardiac output. Data related to the classification group is stored in advance in a memory 172 or the auxiliary storage unit 173. Details of the classification group will be described later.

The hemodynamic parameters can be analyzed as follows, for example. In the following description, the description will be made by exemplifying a case in which the congestion is congestion (body congestion) in the blood vessel and the circulatory failure is peripheral circulatory failure. However, the presently disclosed subject matter is not limited to this case.

First, the threshold value of the venous pressure and the threshold value of the cardiac output are set by the threshold value setting unit 206. The threshold value of the venous pressure is used for determining whether the patient has body congestion (hereinafter, simply referred to as "congestion"), and the threshold value of the cardiac output is used for determining whether the patient has peripheral circulatory failure. These threshold values may be set to a value stored in advance in the memory 172, or can be set by being input by the user through the input device 140. The threshold value of the venous pressure can be set to, for example, 10 mmHg by default. The threshold value of the cardiac output can be set to, for example, 5.5 L/min by default.

When the acquired venous pressure is higher than the threshold value, the hemodynamic parameter analysis unit 204 determines that the patient is more likely to have congestion (for example, can be labeled as "congestion (+)"). On the other hand, when the acquired venous pressure is the threshold value or lower, the hemodynamic parameter analysis unit 204 determines that the patient is less likely to have congestion (for example, can be labeled as "congestion (-)").

When the acquired cardiac output is smaller than the threshold value, the hemodynamic parameter analysis unit 204 determines that the patient is more likely to have peripheral circulatory failure (for example, can be labeled as "peripheral circulatory failure (+)"). On the other hand, when the acquired cardiac output is the threshold value or larger, the hemodynamic parameter analysis unit 204 determines that the patient is less likely to have peripheral circulatory failure (for example, can be labeled as "peripheral circulatory failure (-)").

Next, the analysis result of the hemodynamic parameters of the patient is output (step S103). The display control unit 205 controls the display 151 to display the analysis result of the hemodynamic parameters, for example, a determination result of chances of the congestion and the peripheral circulatory failure. More specifically, when the venous pressure is higher than the threshold value, the display 151 displays that the patient is more likely to have congestion. On the other hand, when the venous pressure is the threshold value or lower, the display 151 displays that the patient is less likely to have congestion. When the cardiac output is smaller than the threshold value, the display 151 displays that the patient is more likely to have peripheral circulatory failure. On the other hand, when the cardiac output is the threshold value or larger, the display 151 displays that the patient is less likely to have peripheral circulatory failure.

As illustrated in FIG. 5, for example, the analysis result of the hemodynamic parameters can be displayed in the matrix form. In FIG. 5, elements [0, 0] to [1, 1] of a 2 × 2 matrix are assigned to the groups I to IV, respectively. For example, the display control unit 205 changes a color of a corresponding region in the groups I to IV according to the values of the venous pressure and the cardiac output to display the above-described classification (the correspondence relation). By displaying the above-described classification, the medical worker can immediately grasp the chance that the patient has the congestion and/or the peripheral circulatory failure, and can understand at a glance the degree of the congestion and the degree of the peripheral circulatory failure based on the threshold values. In an example illustrated in FIG. 5, the region of the matrix of [0, 1] is painted in gray, and it is displayed that the acquired values of the venous pressure and the cardiac output are classified into a group II.

Here, the classification group (the groups I to IV) related to the condition of the patient according to the present embodiment will be described. The groups I to IV are classified based on the set threshold value of the venous pressure and the set threshold value of the cardiac output. The group I is a region in which the acquired venous pressure is lower than the threshold value and the acquired cardiac output is larger than the threshold value. In the group I, it is determined that the condition of the patient is "normal". No treatment is to be performed by the medical worker at a present time point, and it is prompted to prevent and treat a complication while observing the patient. The group II is a region in which the venous pressure is higher than the threshold value and the cardiac output is larger than the threshold value. In the group II, it is determined that the patient is more likely to have congestion and is less likely to have peripheral circulatory failure. It is prompted that the medical worker administers a vasodilator and a diuretic to the patient. The group III is a region in which the venous pressure is lower than the threshold value and the cardiac output is smaller than the threshold value. In the group III, it is determined that the patient is less likely to have congestion and is more likely to have peripheral circulatory failure. It is prompted that the medical worker performs an infusion of a fluid and a blood transfusion to the patient. The group IV is a region in which the venous pressure is higher than the threshold value and the cardiac output is smaller than the threshold value. In the group IV, it is determined that the patient is more likely to have both of congestion and peripheral circulatory failure. It is prompted that the medical worker administers a vasodilator, a diuretic, and cateracolamine to the patient, and uses auxiliary circulation to the patient.

Even if the classification groups are the same, the degree of the congestion may be different depending on magnitude of the venous pressure. Same or similarly, even if the classification groups are the same, the degree of the peripheral circulatory failure may be different depending on magnitude of the cardiac output. The higher the venous pressure, the higher the degree of the congestion is. The lower (the smaller) the cardiac output, the higher the degree of the peripheral circulatory failure is. For example, even when it is determined that a group is the group II or the group IV, the degree of the congestion is higher when the venous pressure is farther from the threshold value, and the degree of the congestion is lower when the venous pressure is closer to the threshold value. Even when it is determined that a group is the group III or the group IV, the degree of the peripheral circulatory failure is higher when the cardiac output is farther from the threshold value, and the degree of the peripheral circulatory failure is lower when the cardiac output is closer to the threshold value.

As illustrated in FIG. 6, for example, the analysis result of the hemodynamic parameters can be displayed in the table form. In FIG. 6, a case in which the venous pressure is "high" indicates a case in which the venous pressure is higher than the threshold value, and a case in which the venous pressure is "low" indicates a case in which the venous pressure is the threshold value or lower. A case in which the cardiac output is "high" indicates a case in which the cardiac output is the threshold value or larger, and a case in which the cardiac output is "low" indicates a case in which the cardiac output is smaller than the threshold value.

For example, when the venous pressure is low and the cardiac output is large, the condition of the patient is classified into the group I, and when both the venous pressure and the cardiac output are high, the condition of the patient is classified into the group II. When both the venous pressure and the cardiac output are low, the condition of the patient is classified into the group III, and when the venous pressure is high and the cardiac output is small, the condition of the patient is classified into the group IV.

For example, the display control unit 205 changes the color of a corresponding region in the groups I to IV according to the values of the venous pressure and the cardiac output to display the above-described classification. In an example in FIG. 6, it is displayed that the acquired values of the venous pressure and the cardiac output correspond to the group IV. Instead of changing the color of the region, a color, a thickness, and the like of characters displayed in the region may be changed.

As illustrated in FIG. 7, a region where the congestion and the peripheral circulatory failure may occur is displayed on the coordinate plane with the central venous pressure and the cardiac output as the coordinate axes. FIG. 7 illustrates a case in which a horizontal axis (an X axis) represents the venous pressure and a vertical axis (a Y axis) represents the cardiac output. However, the vertical axis may represent the venous pressure, and the horizontal axis may represent the cardiac output.

For example, as illustrated in FIG. 8, the display control unit 205 displays, on the coordinate plane, the degrees of the congestion and the peripheral circulatory failure corresponding to the venous pressure and the cardiac output by plotting a marker 10 at a position on the coordinate plane. The position corresponds to the acquired venous pressure and cardiac output. In an example in FIG. 8, the marker 10 indicates that the values of the acquired venous pressure and cardiac output correspond to the group III.

If information on the classification group (for example, the "group I"), the condition ("congestion (+) or the like") of the patient, and a type (the "vasodilator" or the like) of the treatment is displayed at the same time as the marker 10, these pieces of information may overlap with one another, and the display may be difficult to see. In order to prevent the display from being difficult to see, the display of all or a part of the information on the classification group, the condition of the patient, and the type of the treatment can be stopped when the marker 10 is displayed.

The threshold value setting unit 206 can adjust the threshold value of the venous pressure and/or the threshold value of the cardiac output according to at least one of attributes of the patient including sex and age and a state of a disease. For example, as illustrated in FIG. 9, when the patient has a heart disease, the venous pressure is higher than that of a healthy person in a steady state, and the cardiac output is smaller than that of the healthy person in the steady state, the threshold value setting unit 206 can set the threshold value of the venous pressure to a larger value (for example, 15 mmHg) and adjust the threshold value of the cardiac output to a smaller value (for example, 5.0 L/min). Accordingly, it is possible to evaluate whether the current condition of the patient is good or bad by comparing the current condition with the steady state.

Furthermore, as illustrated in FIG. 10, a plurality of threshold values (for example, threshold values 1 to 4) for dividing the coordinate plane into a plurality of regions may be set for the venous pressure and the cardiac output. FIG. 10 illustrates a case in which the axes of the venous pressure and the cardiac output are each divided into three regions by the threshold values 1 to 4. In this case, the coordinate plane is divided into nine regions (groups 1 to 9). The congestion can be labeled as "congestion (1+)" or "congestion (2+)" depending on the degree of the congestion. Same or similarly, the peripheral circulatory failure can be labeled as "peripheral circulatory failure (1+)" or "peripheral circulatory failure (2+)".

In this way, by subdividing the degrees of the congestion and the peripheral circulatory failure, the medical worker can adjust an amount of a medication to be used for the treatment and a medical device to be used according to the degree of the congestion of the patient and the degree of the peripheral circulatory failure of the patient. Therefore, the medical worker can perform finer treatment on the patient.

As described above, in the processing of the flowchart in FIG. 4, the venous pressure and the cardiac output that are calculated based on the physiological information of the patient are acquired, the hemodynamic parameters of the patient are analyzed based on the calculated venous pressure and cardiac output, and the analysis result is output. The measurement control unit 201 controls each measurement apparatus to intermittently acquire the physiological information. The acquisition unit 203 acquires the venous pressure and the cardiac output that are calculated by the calculation unit 202.

### <Operation Example of Display System 100>

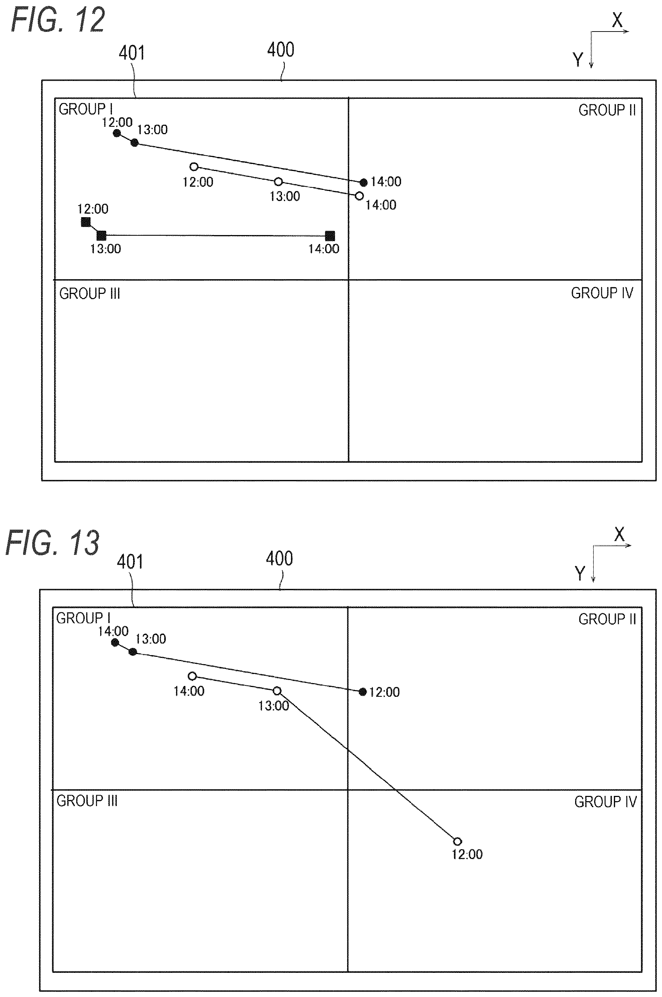

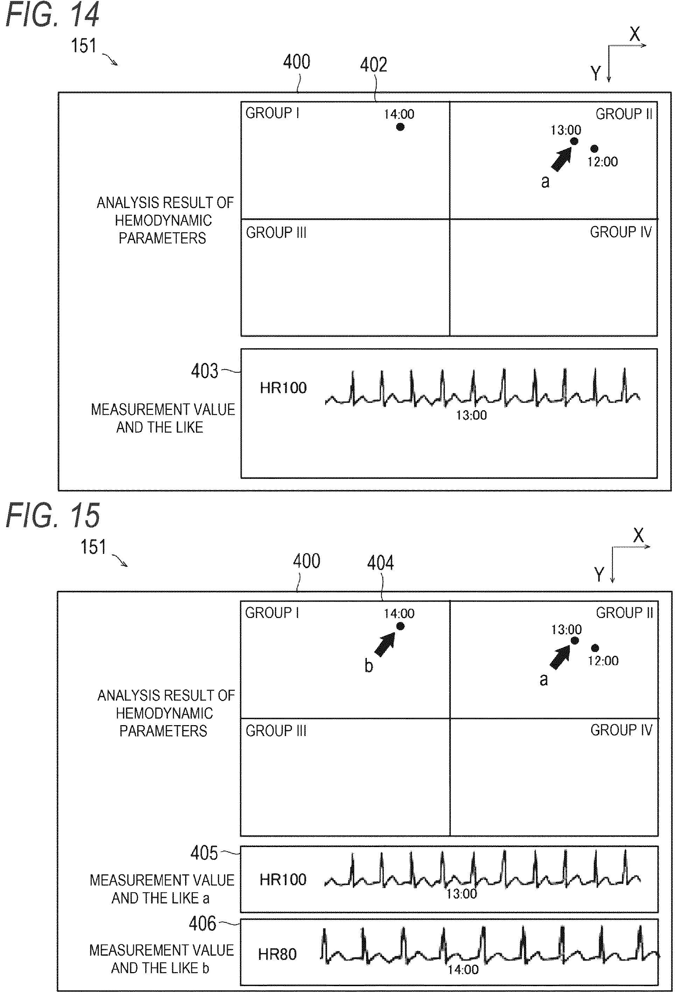

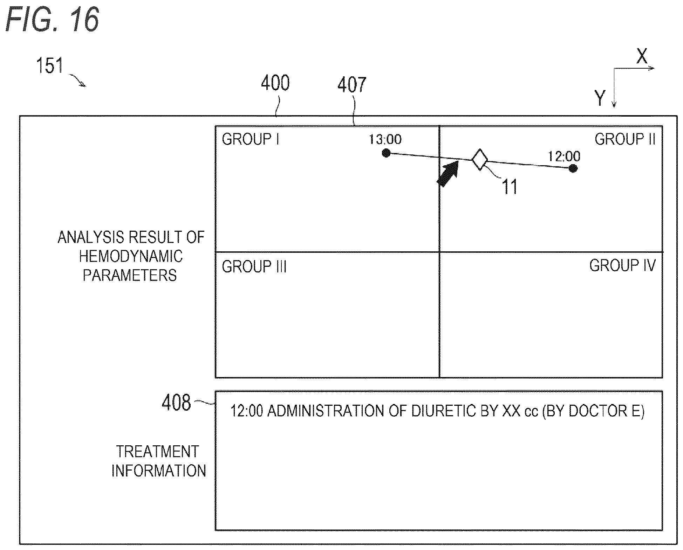

Hereinafter, operation of the display system 100 will be described with reference to FIGS. 11 to 16. FIG. 11 is a schematic diagram illustrating a basic monitor screen of the display apparatus 180. FIGS. 12 and 13 are schematic diagrams illustrating histories of the analysis result of the hemodynamic parameters. FIGS. 14 and 15 are schematic diagrams illustrating a case in which the analysis result of the hemodynamic parameters and measurement values and the like related to the analysis result are displayed together. FIG. 16 is a schematic diagram illustrating a case in which the analysis result of the hemodynamic parameters and treatment information related to the analysis result are displayed together.

The display apparatus 180 is a patient monitor, and is configured to measure, for example, a heart rate, arterial blood pressure, the SpO2, and the like, and to display measurement results on the display 151. As illustrated in FIG. 11, when the display system 100 starts the operation, a basic monitor screen 300 is displayed on the display 151.

The physiological information detected by the blood pressure measurement unit, the electrocardiogram measurement unit 122, and the photoplethysmogram measurement unit described above, the measurement value calculated based on the physiological information, and the like are displayed in real time on a physiological information display region 301 of the basic monitor screen 300. In the present embodiment, in addition to the physiological information, the venous pressure and the cardiac output that are calculated by the calculation unit 202 can also be displayed on the physiological information display region 301.

As a result of analyzing the hemodynamic parameters, when it is determined that an abnormality is more likely to be present in the hemodynamic parameters, for example, when it is determined that the congestion or the peripheral circulatory failure is more likely to occur, an alert message is displayed. In an example illustrated in FIG. 11, when it is determined that the peripheral circulatory failure is more likely to occur, an alert message 302 that "peripheral circulatory failure may occur" is displayed on the display 151.

Further, when it is determined that the abnormality is more likely to be present in the hemodynamic parameters, a screen switching button 303 for shifting to a screen (hereinafter, referred to as a "hemodynamic parameter analysis screen") that displays the analysis result of the hemodynamic parameters can be displayed. Accordingly, the basic monitor screen 300 (a first screen) and the hemodynamic parameter analysis screen (a second screen) can be switched. When the user presses (touches the screen or clicks a pointer) the screen switching button 303, the screen is switched to the hemodynamic parameter analysis screen. Accordingly, the medical worker can easily check the analysis result of the hemodynamic parameters as necessary. The hemodynamic parameter analysis screen may be a screen shown (or similar) in FIGS. 5 to 10 described above, but is more preferably a screen that can check the history as shown in FIG. 12 and the like to be described later.

Positions at which the alert message and the screen switching button are displayed are not limited to a position of the alert message 302 and a position of the screen switching button 303 that are illustrated in FIG. 11. For example, the screen switching button may be displayed at a position indicated by a reference numeral 304, or may be displayed at all times regardless of a possibility of the abnormality in the hemodynamic parameters.

As illustrated in FIG. 12, in a hemodynamic parameter analysis screen 400, the history of the analysis result of the hemodynamic parameters can be displayed. In FIG. 12, the history of the analysis result of the hemodynamic parameters of three patients A, B, C is illustrated at intervals of one hour from 12:00 to 14:00. In this example, the analysis result of the hemodynamic parameters of the plurality of patients is displayed on the same analysis map 401. However, it is generally preferable that the history of the analysis result of one patient is displayed on the one analysis map 401.

The analysis results of the hemodynamic parameters of the patients A, B, C at 12:00, 13:00, and 14:00 are represented by markers "•", "O", and "▪", respectively. For each of the patients A, B, C, the history of the analysis result is represented by the marker at each time and straight lines connecting the markers. Accordingly, the medical worker can grasp at a glance a time course of the analysis result.

For example, the patients A, B are determined to be in the group I (normal) at 12:00 and 13:00, but after that, the condition deteriorates, and at 14:00 (the latest measurement time), the patients A, B are determined to be in the group II (the congestion is more likely to occur). On the other hand, the condition of the patient C deteriorates with the passage of time, but it is determined that the patient C is normal at a time point of 14:00.

For the patients A, B, there is no significant difference in the values of the venous pressure and the cardiac output at the time point of 14:00, distances between the markers from 13:00 to 14:00 are fairly different. That is, the deterioration of the condition of the patient A from 13:00 to 14:00 is rapid, whereas the deterioration of the condition of the patient B from 13:00 to 14:00 is not rapid. At the latest measurement time, there is no big difference in the measured values of the patients A, B, but rates of the deterioration of the conditions are different. Therefore, treatment policies (for example, a medication dosage) for patients A, B made by the medical worker may be different.

When at least one of the venous pressure and the cardiac output changes between different times as described in at least any one of the following conditions (a) to (e), the notification control unit 207 performs control so as to issue an alarm. The notification control unit 207, the input and output interface 174, and the output device 150 function as a notification unit.

### (a) Case in which the venous pressure increases beyond the threshold value

In a case in which the venous pressure increases beyond the threshold value, that is, in a case in which the venous pressure moves from the group I to the group II or the group IV on the analysis map 401 (the coordinate plane), it indicates that the condition of the patient has transitioned from a normal state to a state in which the congestion is more likely to occur.

### (b) Case in which the venous pressure increases beyond a predetermined increase rate

In a case in which the venous pressure increases beyond the predetermined increase rate, that is, in a case in which the venous pressure moves at a high speed with the passage of time in an X direction on the analysis map 401 (the coordinate plane), it indicates that the condition of the patient has deteriorated rapidly. The increase rate is an increase rate of the venous pressure per unit time (for example, 1 min or 1 hr), and corresponds to a horizontal distance (an X-direction component of the distance between the markers) between the markers when the venous pressure is measured at unit time intervals. The predetermined increase rate is not particularly limited, and can be set to, for example, 5 mmHg/1 hr.

### (c) Case in which the cardiac output decreases beyond the threshold value

In a case in which the cardiac output decreases beyond the threshold value, that is, in a case in which the cardiac output moves from the group I to the group III or the group IV on the analysis map 401 (the coordinate plane), it indicates that the condition of the patient has transitioned from the normal state to a state in which the peripheral circulatory failure is more likely to occur.

### (d) Case in which the cardiac output decreases beyond a predetermined decrease rate

In a case in which the cardiac output decreases beyond the predetermined decrease rate, that is, in a case in which the cardiac output moves at a high speed in a Y direction on the analysis map 401 (the coordinate plane), it indicates that the condition of the patient has deteriorated rapidly. The decrease rate is a decrease rate of the cardiac output per unit time (for example, 1 min or 1 hr), and corresponds to a vertical distance (a Y-direction component of the distance between the markers) between the markers when the cardiac output is measured at unit time intervals. The predetermined decrease rate is not particularly limited, and can be set to, for example, 1 L/min/1 hr.

### (e) Case in which the distance between the markers at the latest time and a time immediately before the latest time is a predetermined distance or larger in the coordinate plane

In a case in which the distance between the markers is the predetermined distance or larger, that is, in a case in which the venous pressure and/or the cardiac output moves at a high speed with the passage of time in the X direction and/or the Y direction on the analysis map 401 (the coordinate plane), it indicates that the condition of the patient has deteriorated rapidly. The predetermined distance is stored in advance in the memory 172. However, the predetermined distance may be input and set by the user via the input device 140. Without expanding coordinates, a state in which a change rate of the venous pressure and the cardiac output at a time different from the venous pressure and the cardiac output at a certain time is large may be detected according to any method, and it may be determined that the condition of the patient has deteriorated rapidly.

For example, for any of the patients A, B, since the venous pressure increases beyond the threshold value from 13:00 to 14:00 (corresponding to above-described (a)), the notification control unit 207 controls the input and output interface 174 such that the speaker 152 emits an alarm sound. When the patient C corresponds to the above-described (b), an alarm is issued. Accordingly, when the condition of the patient has deteriorated or when the condition of the patient has suddenly changed, the medical worker can immediately perform the treatment on the patient.

In a case of at least one of the above-described (a) to (e), the measurement control unit 201 increases a frequency of analyzing the hemodynamic parameters of the patient. The measurement control unit 201 also increases a frequency of acquiring the physiological information as the frequency of analyzing the hemodynamic parameters increases. For example, for the patient C having a large increase rate in the venous pressure, the measurement control unit 201 sets the hemodynamic parameter analysis performed at the intervals of one hour to intervals of 20 minutes, and changes the next measurement scheduled to be performed at 15:00 forward to 14:20. By increasing the frequency of analyzing the hemodynamic parameters of the patient, it is possible to quickly notify the medical worker of the deterioration of the condition of the patient.

As illustrated in FIG. 13, the analysis results of the hemodynamic parameters of the patients C, D at 12:00, 13:00, and 14:00 are represented by markers "•" and "O", respectively. For each of the patients C, D, the history of the analysis result is represented by the marker at each time and the straight line connecting the markers.

The patients C, D are determined to be in the group II and the group IV, respectively, at 12:00. However, after that, as a result of medication treatment performed by the medical worker, the condition is improved, and the patients C, D are determined to be in the group I at 13:00. In this way, by displaying the history of the analysis result, the medical worker can easily grasp an effect (a prognosis of the treatment) of the treatment when an abnormality is recognized in the condition of the patient.

The physiological information (a pulse wave waveform, an electrocardiogram waveform, and the like) and/or a measurement value (the arterial blood pressure, the heart rate, a respiration rate, the SpO2, the venous pressure, the cardiac output, and the like) of the patient related to the analysis result can also be displayed together with the analysis result of the hemodynamic parameters. More specifically, as illustrated in FIG. 14, the display control unit 205 displays on the display 151 the analysis result of the hemodynamic parameters and the physiological information and/or the measurement value (hereinafter, referred to as a "measured value and the like") of the patient related to the analysis result side by side. In an example illustrated in FIG. 14, the physiological information (for example, the heartbeat waveform) of the patient is displayed side by side with the analysis map 402 and below the analysis map 402. A plurality of types or a plurality of measurement values and the like may be displayed.

The user can specify the time of the measurement value and the like to be displayed by clicking the time of the analysis result on the analysis map 402 with a pointer (an arrow a in the drawing) or touching the screen. For example, when the user clicks "13:00" on the analysis map 402 with the pointer, the heartbeat waveform in first predetermined time (for example, approximately one minute before and after a designated time) centered on the designated time (13:00) is displayed in a display region 403 of the measured value and the like. An average heart rate (HR100) within this range is displayed. Instead of clicking the time on the analysis map 402 with the pointer, the measurement time (or elapsed time from a reference time of the measurement) may be separately listed on the display 151, and the user can select the time (the elapsed time) to be displayed.

For second predetermined time including the designated time (for example, approximately two hours before and after the designated time), a trend gram of the measured value may be displayed as a waveform (a trend waveform). For example, when the designated time is 13:00, a trend waveform from 11:00 to 15:00 is displayed. The measurement value can be a blood pressure value, the heart rate, the SpO2, and the like. The venous pressure and the cardiac output can also be displayed together with the trend waveform.

In this way, by displaying the measurement value and the like of the patient related to the analysis result together with the analysis result of the hemodynamic parameters, the medical worker can infer a reason why the hemodynamic parameters of the patient have become like the analysis result based on the measured value and the like.

Further, the measurement values and the like at different times can be displayed side by side. More specifically, as illustrated in FIG. 15, the display control unit 205 displays a plurality of measurement values and the like on the display 151 side by side. In an example illustrated in FIG. 15, the physiological information (for example, the heartbeat waveform) of the patient at two different times (13:00 and 14:00) is displayed side by side in an upper-lower direction and below an analysis map 404. A plurality of types or a plurality of measurement values and the like may be displayed in one display region.

The user can specify the time of the measurement value and the like to be displayed by clicking the time of the analysis result on the analysis map 404 with pointers (arrows a, b in the drawing) or touching the screen. For example, when the user clicks "13:00" and "14:00" on the analysis map 404 with the pointers, the heartbeat waveforms in the first predetermined time centered on the designated times (13:00 and 14:00) are displayed in display regions 405, 406 of the measured value and the like, respectively. Average heart rates (HR100 and HR80) within this range are displayed.

In this way, by displaying the measurement values and the like at different times related to the analysis result side by side, the medical worker can easily compare the measurement values and the like at different times. For example, by comparing the measurement values between the case in which it is determined that the condition of the patient is in the group II to the group IV (that is, an abnormality may occur in the hemodynamic parameters) and the case in which it is determined that the condition of the patient is in the group I (that is, the hemodynamic parameters are normal), the medical worker can infer a reason why the hemodynamic parameters become abnormal based on the measurement value and the like by comparing the measurement values and the like between the case in which the hemodynamic parameters of the patient is normal and the case in which an abnormality may occur in the hemodynamic parameters.

However, when a plurality of measurement values and the like are displayed, since the display region of the display 151 is limited, it is not always possible to display all the measurement values and the like related to the analysis result. Therefore, it is possible to set priority order for displaying the measurement values and the like of the patient.

The priority order setting unit 208 is configured to set the priority order for displaying the measurement values and the like of the patient. In terms of the priority order, for example, a default value is stored in advance in the memory 172. In this case, the measurement values and the like are displayed according to, for example, priority order shown in the following table 1.

**[Table 1]**

| Priority order | Measurement value and the like |
|---|---|
| High | Blood pressure value, heart rate, venous pressure, and cardiac output |
| Medium | Electrocardiogram waveform |
| Low | Respiration rate |

The priority order setting unit 208 can also set the priority order based on input of the user. For example, the user can use the input device 140 to input the priority order of the measurement values and the like in the table 1 with characters of "high", "medium", and "low", numerical values, and the like.

The priority order setting unit 208 can also set the priority order according to the classification group determined by the hemodynamic parameter analysis unit 204. For example, when the condition is improved from the state (the group III or the group IV) in which the contractile function of the heart is lowered to normal (the group I), the priority order of the heart rate and the cardiac output is set to "high". On the other hand, when the condition is improved from the state (the group II or the group IV) in which the amount of circulating blood is decreased to normal (the group I), the priority order of the blood pressure value and the venous pressure is set to "high". The display control unit 205 selects the measurement value and the like of the patient according to the priority order set by the priority order setting unit 208, and displays the selected measurement value and the like on the display 151. Accordingly, it is possible to effectively display the measurement value and the like required by the medical worker in the limited display region of the display 151.

Information (hereinafter, referred to as "treatment information") related to the treatment of the medical worker can also be displayed in relation to the analysis result. The treatment information may be directly input to the display apparatus 180 by the medical worker at a time of performing the treatment, or information managed by various systems (for example, an intraoperative management system) on the communication network may be acquired.

When the condition of the patient is changed (improved or deteriorated) as a result of the performed treatment, it is necessary for the medical worker who has performed the treatment or a member of a medical team involved in the treatment of the patient to know what treatment has resulted in the change in the condition of the patient. For example, when a medication is administered to the patient, it is necessary to know what kind of medication has been administered and an amount of the medication that has been administered.

On the other hand, the display control unit 205 may display all pieces of treatment information related to the analysis result of the hemodynamic parameter analysis of the target patient on the display 151. However, displaying a lot of pieces of information in the limited display region may be complicated. Therefore, the display control unit 205 can also select and display only the treatment performed in a period desired by the user. For example, as illustrated in FIG. 16, the user can specify a period during which the treatment information is displayed by clicking a straight line connecting two markers indicating the analysis results at different times on the analysis map 407 or an icon 11 of a rhombus ("◊") on the straight line or touching the screen.

For example, it is determined that the condition of the patient is in the group II at 12:00. However, since a doctor E administers a diuretic to the patient by XX [cc], the condition is improved, and it is determined that the condition is in the group I at 13:00. When the user clicks the straight line between the marker of "12:00" and the marker of "13:00" on the analysis map 407 or the icon 11 with the pointer of the arrow, the treatment information of the designated period is displayed in a display region 408 of the treatment information. In the display region 408 of the treatment information, "12:00 administration of diuretic by XX cc (by doctor E)" is displayed. That is, it is displayed that the doctor E has administered the diuretic to the patient at 12:00. The treatment information may be displayed on the analysis map 407. In an example illustrated in FIG. 16, the treatment information can be displayed in a vicinity of the icon 11 in a form of being put in a balloon.

In this way, the treatment information of the period desired by the user is displayed in relation to the analysis result. Therefore, when the condition of the patient is changed (improved or deteriorated) as a result of the performed treatment, the medical worker can easily check what treatment has resulted in the change in the condition of the patient.

### <Method for Calculating Venous Pressure>

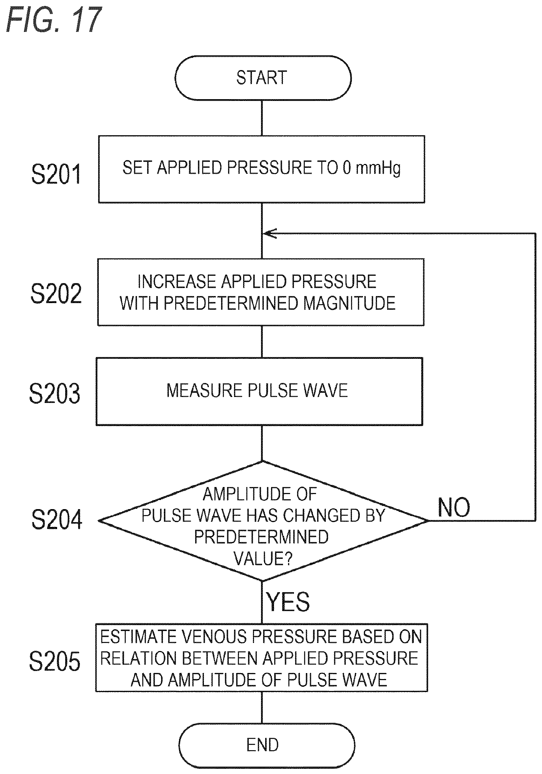

FIG. 17 is a flowchart illustrating a processing procedure for calculating the venous pressure. The processing of the flowchart in the drawing is implemented by the CPU 171 executing the analysis program.

When the pulse sensor 131 is attached to the left finger of the patient, the cuff 111 is preferably attached to a right arm. However, the presently disclosed subject matter is not limited thereto. Since an average venous pressure measured when a posture of the cuff 111 and the heart having the same height is adopted can be considered as the central venous pressure, the central venous pressure can be measured by a blood pressure measurement unit according to the present embodiment by adopting this posture.

First, the cuff 111 is attached to an upper arm in a vicinity of an axilla of the patient, and the measurement control unit 201 sets applied pressure (cuff pressure) applied by the cuff 111 to the upper arm in the vicinity of the axilla to 0 mmHg (step S201).

Next, the applied pressure is increased with a predetermined increase width (step S202), and the cuff pulse wave is measured by the cuff 111 and the pressure sensor 114 (step S203). In the processing of increasing the pressure with the predetermined pressure increase width, the cuff pulse wave is measured. A pulse wave amplitude changes according to the applied pressure. Based on a principle of an oscillometric method, the pulse wave amplitude becomes the maximum when the cuff pressure becomes equal to the average blood pressure. The measurement of the cuff pulse wave to be performed while increasing the applied pressure is repeated until an amplitude of the cuff pulse wave is changed by a predetermined value (step S204: NO).

When the amplitude of the cuff pulse wave has changed by the predetermined value (S204: YES), the measurement is terminated, and the venous pressure is estimated based on relation between the applied pressure and the change in the amplitude of the cuff pulse wave (step S205). In step S204, when the applied pressure reaches predetermined maximum applied pressure, the measurement is terminated from a viewpoint of safety.

In an example in FIG. 17, a case has been described in which the cuff pulse wave is measured while the applied pressure is increased from 0 mmHg, and the venous pressure is estimated based on the applied pressure. At the applied pressure, the amplitude of the cuff pulse wave has changed by the predetermined value. However, the presently disclosed subject matter is not limited to this case. The cuff pulse wave may be measured while lowering the applied pressure from predetermined pressure, and the venous pressure may be estimated based on the applied pressure at which the amplitude of the cuff pulse wave has changed by the predetermined value.

In this way, in the present embodiment, the calculation unit 202 applies the cuff pressure to the upper arm in the vicinity of the axilla within a range equal to or smaller than the maximum applied pressure, and changes the applied cuff pressure to obtain the cuff pressure at which the amplitude of the cuff pulse wave detected by the cuff has changed by the predetermined value. That is, the calculation unit 202 estimates the average venous pressure by detecting a change in the amplitude of the pulse pressure caused by a pressure closure of a vein when the cuff pressure reaches the average venous pressure.

In this way, in the present embodiment, the venous pressure can be estimated non-invasively with a simple configuration including one cuff 111 and one pressure sensor 114. The method for calculating the venous pressure is not limited to the above-described method, and for example, methods described in a first embodiment to a sixth embodiment of Japanese Patent No. 5694032 can also be used.

### <Method for Calculating Cardiac Output>

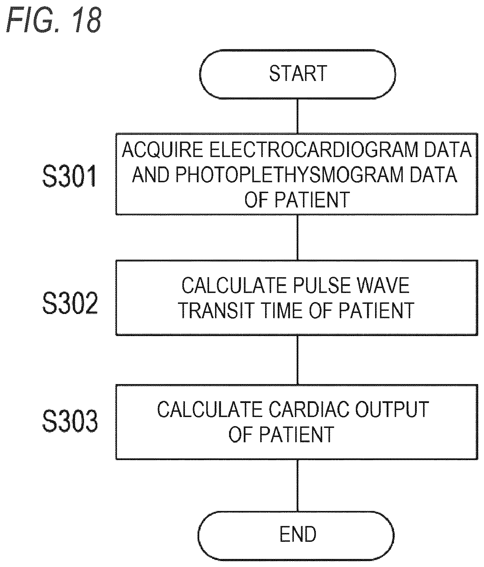

FIG. 18 is a flowchart illustrating a processing procedure for calculating the cardiac output. The processing of the flowchart in the drawing is implemented by the CPU 171 executing the analysis program.

In the present embodiment, a method is exemplified for calculating the cardiac output based on pulse wave transit time (hereinafter, also referred to as "PWTT") indicating a time interval from a peak point of a predetermined R wave on the electrocardiogram to a rising point of a predetermined pulse wave waveform. The predetermined pulse wave waveform appears next to the predetermined R wave.

As illustrated in FIG. 18, electrocardiogram data and photoplethysmogram data of the patient are acquired (step S301). The measurement control unit 201 controls an electrocardiogram measurement apparatus 120 to measure the electrocardiogram of the patient and to transmit the electrocardiogram data to the calculation unit 202. The measurement control unit 201 controls a photoplethysmogram measurement unit to measure the photoplethysmogram of the patient and to transmit the photoplethysmogram data to the calculation unit 202.

Next, the pulse wave transit time of the patient is calculated (step S302). The calculation unit 202 measures the pulse wave transit time based on the electrocardiogram data and the photoplethysmogram data. More specifically, the calculation unit 202 specifies a time of the peak point of the predetermined R wave based on the electrocardiogram data, and specifies a time of the rising point of the predetermined pulse wave waveform that appears next to the predetermined R wave based on the photoplethysmogram data. Then, the calculation unit 202 measures the pulse wave transit time by calculating the time interval between the time of the rising point of the predetermined pulse wave waveform and the time of the peak point of the predetermined R wave. The calculation unit 202 calculates the pulse wave transit time for every predetermined time (for example, one second).

Next, the cardiac output of the patient is calculated (step S303). The calculation unit 202 estimates the cardiac output of the patient based on the calculated pulse wave transit time and the heart rate. It is known that the cardiac output of the patient can be estimated using the following Equation (1) (for example, see JP2005-312947A).$esCCO = K \times \left(α\times PWTT+β\right) \times HR$

Here, estimated continuous cardiac output (esCCO) represents the estimated (calculated) cardiac output of the patient, and HR represents the heart rate of the patient. K, α, and β are unique coefficients that are set for each patient.

In this way, the cardiac output can be estimated non-invasively based on the electrocardiogram data and the photoplethysmogram data.

As described above, according to the physiological information display apparatus 180 in the present embodiment, the hemodynamic parameters of the patient are analyzed based on the venous pressure and the cardiac output that are calculated based on the physiological information of the patient. Therefore, the hemodynamic parameters of the patient can be estimated while reducing the burden on the patient for measurement.

As described above, in the embodiment, the physiological information display system 100, the physiological information display apparatus 180, and the analysis program according to the presently disclosed subject matter have been described. However, it is needless to say that the presently disclosed subject matter can be appropriately added, modified, and omitted by those skilled in the art within the scope of the technical idea.

For example, in the above-described embodiment, the markers are plotted at the positions corresponding to the measurement values of the venous pressure and the cardiac output on the analysis map or the coordinate plane, and the measurement time is displayed, so that a time course (a history) of the measurement value is shown. However, a method for displaying the time course of the measurement value is not limited thereto.

In the above-described example, a case has been described in which the axis of each of the venous pressure and the cardiac output is divided into a plurality of regions by the threshold values on the analysis map or the coordinate plane. However, the presently disclosed subject matter is not limited to this case, and the line representing the threshold value and/or the region may not be displayed. That is, the display control unit 205 displays the venous pressure and the cardiac output that are acquired by the acquisition unit 203 on the display on a coordinate plane in which the venous pressure and the cardiac output are taken as coordinate axes. This is because, by checking only the position of the marker plotted on the analysis map or the coordinate plane without being conscious of the positional relation between the threshold value or the region and the marker, the medical worker can determine the degree of the congestion and the degree of the circulatory failure.

In the above-described example, a case has been described in which, based on the venous pressure and the cardiac output, the hemodynamic parameters of the patient are analyzed or information on the hemodynamic parameters is displayed. However, the presently disclosed subject matter is not limited to this case. For example, the cardiac index may be calculated based on the calculated cardiac output, and based on the venous pressure and the cardiac index, the hemodynamic parameters of the patient may be analyzed or the information on the hemodynamic parameters may be displayed. The cardiac index is an index representing the cardiac function converted into an amount per body surface area by dividing the cardiac output of the patient by a body surface area so as to correct a physical difference of the patient. The cardiac index may be directly input by the user through the input device 140, or may be estimated based on a height and a weight of the patient that are input in advance.

In the above-described example, the venous pressure is calculated non-invasively based on the pulse wave acquired from the subject and the applied pressure applied to the subject using the cuff. However, the presently disclosed subject matter is not limited thereto. That is, the physiological information display apparatus 180 may non-invasively calculate the venous pressure based on a signal acquired by a sensor that is in contact with or close to a body surface of the subject.

For example, a sensor including a light source and a photodetector may be provided at a neck of the subject, and the venous pressure may be estimated by performing operation using near infrared spectroscopy (NIRS) on an optical signal acquired by the sensor.

A unit and a method for performing various types of processing in the physiological information display apparatus 180 according to the above-described embodiment can be implemented by a dedicated hardware circuit or a programmed computer. The program may be provided by a computer-readable recording medium such as a compact disc read only memory (CD-ROM), or may be provided online via a network such as the Internet. In this case, a program recorded in the computer-readable recording medium is normally transferred to and stored in a storage unit such as a hard disk. The above-described program may be provided as independent application software, or may be incorporated into software of the physiological information display apparatus 180 as one function of the apparatus.

## Claims

1. A hemodynamic parameter analysis apparatus (180) comprising:
an acquisition unit (203) configured to acquire venous pressure and cardiac output that are calculated based on physiological information of a subject;
a hemodynamic parameter analysis unit (204) configured to analyze hemodynamic parameters of the subject based on the venous pressure and the cardiac output that are acquired by the acquisition unit (203), and
a threshold value setting unit (206) configured to set a threshold value for venous pressure and cardiac output
wherein the hemodynamic parameter analysis unit (204) is configured to estimate a degree of congestion of the subject to be estimated based on the venous pressure and a degree of circulatory failure of the subject to be estimated based on the cardiac output, and
wherein the hemodynamic parameter analysis unit (204) is configured to determine a classification corresponding to the degree of congestion of the subject and the degree of circulatory failure of the subject based on a classification group related to congestion and circulatory failure, the classification group being classified based on the threshold value of venous pressure and the threshold value of cardiac output.

2. The hemodynamic parameter analysis apparatus (180) according to claim 1,
wherein the hemodynamic parameter analysis unit (204) is configured to analyze at least one of congestion of the subject and circulatory failure of the subject based on the venous pressure and the cardiac output.

3. The hemodynamic parameter analysis apparatus (180) according to claim 1 or 2,
wherein the threshold value setting unit (206) is configured to adjust a threshold value of venous pressure and/or a threshold value of cardiac output based on at least one of an attribute of the subject including sex and age and a state of a disease of the subject.

4. The hemodynamic parameter analysis apparatus (180) according to any one of claims 1 to 3, further comprising:
a notification unit (207),
wherein the acquisition unit (203) is configured to acquire venous pressure and cardiac output of the subject at different times, and
wherein the notification unit (207) is configured to issue an alarm when at least one of conditions (a) to (d) is satisfied during the different times, the conditions (a) to (d) including:
(a) a condition that venous pressure acquired by the acquisition unit exceeds a threshold value;
(b) a condition that the venous pressure exceeds a predetermined increase rate;
(c) a condition that cardiac output acquired by the acquisition unit decreases beyond a threshold value; and
(d) a condition that the cardiac output decreases beyond a predetermined decrease rate.

5. The hemodynamic parameter analysis apparatus (180) according to any one of claims 1 to 4, further comprising:
a physiological information acquisition unit configured to acquire physiological information of the subject,
wherein the acquisition unit (203) is configured to acquire venous pressure and cardiac output of the subject at different times, and
wherein the physiological information acquisition unit (201) is configured to increase a frequency of acquiring physiological information of the subject when at least one of conditions (a) to (d) is satisfied during the different times, the conditions (a) to (d) including:
(a) a condition that venous pressure acquired by the acquisition unit exceeds a threshold value;
(b) a condition that the venous pressure exceeds a predetermined increase rate;
(c) a condition that cardiac output acquired by the acquisition unit decreases beyond a threshold value; and
(d) a condition that the cardiac output decreases beyond a predetermined decrease rate.

6. The hemodynamic parameter analysis apparatus (180) according to any one of claims 1 to 5, further comprising
an output unit (150) configured to output an analysis result of hemodynamic parameters of the subject analyzed by the hemodynamic parameter analysis unit (204).

7. The hemodynamic parameter analysis apparatus (180) according to claim 6,
wherein the output unit (150) includes a display (151) configured to display the analysis result, and
wherein the display (151) is configured to display an analysis result of hemodynamic parameters of the subject on a coordinate plane with venous pressure and cardiac output as coordinate axes.

8. The hemodynamic parameter analysis apparatus (180) according to claim 1,
further comprising a display (151) configured to display venous pressure and cardiac output that are acquired by the acquisition unit (203) on a coordinate plane with venous pressure and cardiac output as coordinate axes;
wherein the acquisition unit (203) is configured to calculate venous pressure non-invasively based on a signal acquired by a sensor that is in contact with or close to a body surface of a subject, and to acquire cardiac output non-invasively calculated based on a heart rate of the subject and pulse wave transit time obtained based on a pulse wave.

9. The hemodynamic parameter analysis apparatus (180) according to claim 8,
wherein the acquisition unit (203) is configured to non-invasively calculate the venous pressure based on a pulse wave acquired from the subject and applied pressure applied to the subject.

10. The hemodynamic parameter analysis apparatus (180) according to any one of claims 1 to 9, further comprising
a calculation unit (202) configured to calculate venous pressure and cardiac output of the subject based on physiological information of the subject,
wherein the acquisition unit (203) is configured to acquire venous pressure and cardiac output that are calculated by the calculation unit (202).

11. The hemodynamic parameter analysis apparatus (180) according to claim 7 or 8,
wherein the display (151) is configured to display a degree of congestion and peripheral circulatory failure in each region divided by the threshold value on the coordinate plane.

12. The hemodynamic parameter analysis apparatus (180) according to claim 11,
wherein the threshold value setting unit (206) is configured to respectively set a threshold value of venous pressure and a threshold value of cardiac output for venous pressure and cardiac output so as to divide the coordinate plane into four regions, and
wherein the display (151) is configured to
display, in a region where venous pressure on the coordinate plane is larger than the threshold value of venous pressure, that a possibility of congestion is present, and
display, in a region where cardiac output on the coordinate plane is smaller than the threshold value of cardiac output, that a possibility of peripheral circulatory failure is present.

13. The hemodynamic parameter analysis apparatus (180) according to any one of claims 7, 8, 9, 11 and 12,
wherein the acquisition unit (203) is configured to acquire venous pressure and cardiac output of the subject at different times, and
wherein the display (151) is configured to display a history of venous pressure and cardiac output at the different times by plotting a marker at each position of the coordinate plane corresponding to the venous pressure and the cardiac output.

14. The hemodynamic parameter analysis apparatus (180) according to claim 13, further comprising
a notification unit (207),
wherein the notification unit (207) is configured to issue an alarm when at least one of conditions (a) to (e) is satisfied during the different times, the conditions (a) to (e) including:
(a) a condition that venous pressure acquired by the acquisition unit exceeds a threshold value;
(b) a condition that the venous pressure exceeds a predetermined increase rate;
(c) a condition that cardiac output acquired by the acquisition unit decreases beyond a threshold value;
(d) a condition that the cardiac output decreases beyond a predetermined decrease rate; and
(e) a condition that a distance between markers at a latest time and a time immediately before the latest time is a predetermined distance or larger.

15. The hemodynamic parameter analysis apparatus (180) according to claim 13, further comprising
a physiological information acquisition unit (201) configured to acquire physiological information of the subject,
wherein the physiological information acquisition unit (201) is configured to increase a frequency of acquiring physiological information of the subject when at least one of conditions (a) to (e) is satisfied during the different times, the conditions (a) to (e) including:
(a) a condition that venous pressure acquired by the acquisition unit exceeds a threshold value;
(b) a condition that the venous pressure exceeds a predetermined increase rate;
(c) a condition that cardiac output acquired by the acquisition unit decreases beyond a threshold value;
(d) a condition that the cardiac output decreases beyond a predetermined decrease rate; and
(e) a condition that a distance between markers at a latest time and a time immediately before the latest time is a predetermined distance or larger.

16. The hemodynamic parameter analysis apparatus (180) according to claim 12,
wherein the display (151) is configured to
switch between a first screen configured to display physiological information of the subject and/or a measurement value measured based on the physiological information and a second screen configured to display an analysis result of hemodynamic parameters of the subject, and
display, when the first screen is displayed, that a possibility of congestion is present on the first screen in a case in which venous pressure acquired by the acquisition unit exceeds the threshold value of venous pressure, and that a possibility of peripheral circulatory failure is present on the first screen in a case in which cardiac output acquired by the acquisition unit exceeds the threshold value of cardiac output.

17. The hemodynamic parameter analysis apparatus (180) according to any one of claims 7, 8, 9 and 11 to 15,
wherein the display (151) is configured to display an analysis result of hemodynamic parameters of the subject together with physiological information of the subject and/or a measurement value measured based on the physiological information, the physiological information and the measurement value being related to the analysis result.

18. The hemodynamic parameter analysis apparatus (180) according to claim 17,
wherein the acquisition unit (203) is configured to acquire venous pressure and cardiac output of the subject at different times, and
wherein the display (151) is configured to display an analysis result of hemodynamic parameters of the subject at the different times together with physiological information of the subject and/or a measurement value measured based on the physiological information, the physiological information and the measurement value being related to each analysis result.

19. The hemodynamic parameter analysis apparatus (180) according to claim 18, further comprising
a priority order setting unit (208) configured to set priority order for displaying physiological information of the subject and/or a measured value measured based on the physiological information,
wherein the display (151) is configured to display physiological information of the subject and/or a measured value measured based on the physiological information according to priority order set by the priority order setting unit.

20. The hemodynamic parameter analysis apparatus (180) according to claim 13,
wherein the display (151) is configured to display, when acquired venous pressure decreases or increases beyond a threshold value or when acquired cardiac output decreases or increases beyond a threshold value during the different times due to treatment performed by a medical worker for the subject, information related to the treatment performed by the medical worker and a time at which the treatment is performed.

21. A hemodynamic parameter analysis program for causing a computer to execute processing comprising:
a first step of acquiring venous pressure and cardiac output that are calculated based on physiological information of a subject;
a second step of analyzing hemodynamic parameters of the subject based on the venous pressure and the cardiac output that are acquired in the first step,
setting a threshold value for venous pressure and cardiac output,
estimating a degree of congestion of the subject to be estimated based on the venous pressure and a degree of circulatory failure of the subject to be estimated based on the cardiac output, and determining a classification corresponding to the degree of congestion of the subject and the degree of circulatory failure of the subject based on a classification group related to congestion and circulatory failure, the classification group being classified based on the threshold value of venous pressure and the threshold value of cardiac output.

22. The hemodynamic parameter analysis program for causing a computer to execute processing according to claim 21, comprising:
in the first step, non-invasively calculating venous pressure based on a signal acquired by a sensor that is in contact with or close to a body surface of a subject, and acquiring cardiac output non-invasively calculated based on a heart rate of the subject and pulse wave transit time obtained based on a pulse wave; and
in the second step, displaying the venous pressure and the cardiac output that are acquired in the first step on a coordinate plane with venous pressure and cardiac output as coordinate axes.

## Patentansprüche

1. Vorrichtung für hämodynamische Parameteranalyse (180), die umfasst:
eine Erfassungs-Einheit (203), die so ausgeführt ist, dass sie Venendruck sowie Herzminutenvolumen erfasst, die auf Basis physiologischer Informationen eines Patienten berechnet werden;
eine Einheit (204) für hämodynamische Parameteranalyse, die so ausgeführt ist, dass sie hämodynamische Parameter des Subjekts auf Basis des Venendrucks sowie des Herzminutenvolumens analysiert, die durch die Erfassungs-Einheit (203) erfasst werden, und
eine Einheit (206) zum Festlegen eines Schwellenwertes, die so ausgeführt ist, dass sie einen Schwellenwert für Venendruck und Herzminutenvolumen festlegt,
wobei die Einheit (204) für hämodynamische Parameteranalyse so ausgeführt ist, dass sie einen Grad von Kongestion des Patienten, der auf Basis des Venendrucks geschätzt wird, sowie einen Grad von Kreislaufversagen des Patienten schätzt, der auf Basis des Herzminutenvolumens geschätzt wird, und
wobei die Einheit (204) für hämodynamische Parameteranalyse, so ausgeführt ist, dass sie eine Klassifizierung, die dem Grad an Kongestion des Patienten sowie dem Grad an Kreislaufversagen des Patienten entspricht, auf Basis einer Klassifizierungsgruppe bestimmt, die sich auf Kongestion und Kreislaufversagen bezieht, wobei die Klassifizierungsgruppe auf Basis des Schwellenwertes von Venendruck sowie des Schwellenwertes von Herzminutenvolumen klassifiziert wird.

2. Vorrichtung (180) für hämodynamische Parameteranalyse nach Anspruch 1,
wobei die Einheit (204) für hämodynamische Parameteranalyse so ausgeführt ist, dass sie Kongestion des Patienten oder/und Kreislaufversagen des Patienten auf Basis des Venendrucks sowie des Herzminutenvolumens analysiert.

3. Vorrichtung (180) für hämodynamische Parameteranalyse nach Anspruch 1 oder 2,
wobei die Einheit (206) zum Festlegen eines Schwellenwertes so ausgeführt ist, dass sie einen Schwellenwert von Venendruck und/oder einen Schwellenwert von Herzminutenvolumen basierend auf einem Attribut des Patienten einschließlich Geschlecht und Alter oder/und einem Krankheitszustand des Patienten anpasst.

4. Vorrichtung (180) für hämodynamische Parameteranalyse nach einem der Ansprüche 1 bis 3, die des Weiteren umfasst:
eine Benachrichtigungs-Einheit (207),
wobei die Erfassungs-Einheit (203) so ausgeführt ist, dass sie Venendruck sowie Herzminutenvolumen des Patienten zu unterschiedlichen Zeiten erfasst, und
wobei die Benachrichtigungs-Einheit (207) so ausgeführt ist, dass sie eine Warnmeldung ausgibt, wenn wenigstens eine der folgenden Bedingungen (a) bis (d) zu den unterschiedlichen Zeiten erfüllt ist, wobei die Bedingungen (a) bis (d) einschließen:
(a) eine Bedingung dahingehend, dass durch die Erfassungs-Einheit erfasster Venendruck einen Schwellenwert überschreitet;
(b) eine Bedingung dahingehend, dass der Venendruck eine vorgegebene Zunahmerate überschreitet;
(c) eine Bedingung dahingehend, dass durch die Erfassungs-Einheit erfasstes Herzminutenvolumen über einen Schwellenwert hinaus abnimmt; und
(d) eine Bedingung dahingehend, dass das Herzminutenvolumen über eine vorgegebene Abnahmerate hinaus abnimmt.

5. Vorrichtung (180) für hämodynamische Parameteranalyse nach einem der Ansprüche 1 bis 4, die des Weiteren umfasst:
eine Einheit für Erfassung physiologischer Informationen, die so ausgeführt ist, dass sie physiologische Informationen des Patienten erfasst,
wobei die Erfassungs-Einheit (203) so ausgeführt ist, dass sie Venendruck sowie Herzminutenvolumen des Patienten zu unterschiedlichen Zeiten erfasst, und
wobei die Einheit (201) für Erfassung physiologischer Informationen so ausgeführt ist, dass sie eine Frequenz beim Erfassen physiologischer Informationen des Patienten erhöht, wenn wenigstens eine der folgenden Bedingungen (a) bis (d) zu den unterschiedlichen Zeiten erfüllt ist, wobei die Bedingungen (a) bis (d) einschließen:
(a) eine Bedingung dahingehend, dass durch die Erfassungs-Einheit erfasster Venendruck einen Schwellenwert überschreitet;
(b) eine Bedingung dahingehend, dass der Venendruck eine vorgegebene Zunahmerate überschreitet;
(c) eine Bedingung dahingehend, dass durch die Erfassungs-Einheit erfasstes Herzminutenvolumen über einen Schwellenwert hinaus abnimmt; und
(d) eine Bedingung dahingehend, dass das Herzminutenvolumen über eine vorgegebene Abnahmerate hinaus abnimmt.

6. Vorrichtung (180) für hämodynamische Parameteranalyse nach einem der Ansprüche 1 bis 5, die des Weiteren umfasst:
eine Ausgabe-Einheit (150), die so ausgeführt ist, dass sie ein Ergebnis von Analyse durch die Einheit (204) für hämodynamische Parameteranalyse analysierter hämodynamischer Parameter des Patienten ausgibt.

7. Vorrichtung (180) für hämodynamische Parameteranalyse nach Anspruch 6,
wobei die Ausgabe-Einheit (150) eine Anzeigeeinrichtung (151) aufweist, die zum Anzeigen des Ergebnisses von Analyse ausgeführt ist, und
wobei die Anzeigeeinrichtung (151) so ausgeführt ist, dass sie ein Ergebnis von Analyse hämodynamischer Parameter des Patienten auf einer Koordinatenebene mit Venendruck und Herzminutenvolumen als Koordinatenachsen anzeigt.

8. Vorrichtung (180) für hämodynamische Parameteranalyse nach Anspruch 1,
die des Weiteren eine Anzeigeeinrichtung (151) umfasst, die so ausgeführt ist, dass sie Venendruck und Herzminutenvolumen, die durch die Erfassungs-Einheit (203) erfasst werden, auf einer Koordinatenebene mit Venendruck und Herzminutenvolumen als Koordinatenachsen anzeigt;
wobei die Erfassungs-Einheit (203) so ausgeführt ist, dass sie Venendruck nicht-invasiv auf Basis eines Signals berechnet, das von einem Sensor erfasst wird, der sich in Kontakt mit oder nahe an einer Körperoberfläche des Patienten befindet, und Herzminutenvolumen nicht-invasiv, berechnet auf Basis einer Pulszahl des Patienten sowie Pulswellenlaufzeit, erfasst, die auf Basis einer Pulswelle ermittelt wird.

9. Vorrichtung (180) für hämodynamische Parameteranalyse nach Anspruch 8,
wobei die Erfassungs-Einheit (203) so ausgeführt ist, dass sie den Venendruck auf Basis einer von dem Patienten erfassten Pulswelle und auf den Patienten ausgeübten Drucks nicht-invasiv berechnet.

10. Vorrichtung (180) für hämodynamische Parameteranalyse nach einem der Ansprüche 1 bis 9, die des Weiteren umfasst:
eine Berechnungs-Einheit (202), die so ausgeführt ist, dass sie Venendruck sowie Herzminutenvolumen des Patienten auf Basis physiologischer Informationen des Patienten berechnet,
wobei die Erfassungs-Einheit (203) so ausgeführt ist, dass sie Venendruck sowie Herzminutenvolumen erfasst, die durch die Berechnungs-Einheit (202) berechnet werden.

11. Vorrichtung (180) für hämodynamische Parameteranalyse nach Anspruch 7 oder 8,
wobei die Anzeigeeinrichtung (151) so ausgeführt ist, dass sie einen Grad an Kongestion und peripherem Kreislaufversagen in jedem durch den Schwellenwert auf der Koordinatenebene geteilten Bereich anzeigt.

12. Vorrichtung (180) für hämodynamische Parameteranalyse nach Anspruch 11;
wobei die Einheit (206) zum Einstellen des Schwellenwertes so ausgeführt ist, dass sie jeweils einen Schwellenwert von Venendruck sowie einen Schwellenwert von Herzminutenvolumen so festlegt, dass die Koordinatenebene in vier Bereiche unterteilt wird, und
wobei die Anzeigeeinrichtung (151) so ausgeführt ist, dass sie
in einem Bereich, in dem Venendruck auf der Koordinatenebene über dem Schwellenwert für Venendruck liegt, anzeigt, dass eine Möglichkeit von Kongestion vorliegt, und
in einem Bereich, in dem Herzminutenvolumen auf der Koordinatenebene unter dem Schwellenwert für Herzminutenvolumen liegt, anzeigt, dass eine Möglichkeit von peripherem Kreislaufversagen vorliegt.

13. Vorrichtung (180) für hämodynamische Parameteranalyse nach einem der Ansprüche 7, 8, 9, 11 und 12,
wobei die Erfassungs-Einheit (203) so ausgeführt ist, dass sie Venendruck sowie Herzminutenvolumen des Patienten zu unterschiedlichen Zeiten erfasst, und
wobei die Anzeigeeinrichtung (151) so ausgeführt ist, dass sie einen Verlauf von Venendruck und Herzminutenvolumen zu den unterschiedlichen Zeiten anzeigt, indem sie eine Markierung an jeder Position der Koordinatenebene darstellt, die dem Venendruck und den Herzminutenvolumen entspricht.

14. Vorrichtung (180) für hämodynamische Parameteranalyse nach Anspruch 13, die des Weiteren umfasst:
eine Benachrichtigungs-Einheit (207,
wobei die Benachrichtigungs-Einheit (207) so ausgeführt ist, dass sie eine Warnmeldung ausgibt, wenn wenigstens eine der folgenden Bedingungen (a) bis (e) zu den unterschiedlichen Zeiten erfüllt ist, wobei die Bedingungen (a) bis (e) einschließen:
(a) eine Bedingung dahingehend, dass durch die Erfassungs-Einheit erfasster Venendruck einen Schwellenwert überschreitet;
(b) eine Bedingung dahingehend, dass der Venendruck eine vorgegebene Zunahmerate überschreitet;
(c) eine Bedingung dahingehend, dass durch die Erfassungs-Einheit erfasstes Herzminutenvolumen über einen Schwellenwert hinaus abnimmt;
(d) eine Bedingung dahingehend, dass das Herzminutenvolumen über eine vorgegebene Abnahmerate hinaus abnimmt; und
(e) eine Bedingung dahingehend, dass ein Abstand zwischen Markierungen zu einer spätesten Zeit und zu einer Zeit unmittelbar vor der spätesten Zeit ein vorgegebener oder größerer Abstand ist.

15. Vorrichtung (180) für hämodynamische Parameteranalyse nach Anspruch 13, die des Weiteren umfasst:
eine Einheit (201) für Erfassung physiologischer Informationen, die so ausgeführt ist, dass sie physiologische Informationen des Patienten erfasst,
wobei die Einheit (201) für Erfassung physiologischer Informationen so ausgeführt ist, dass sie eine Frequenz beim Erfassen physiologischer Informationen des Patienten erhöht, wenn wenigstens eine der folgenden Bedingungen (a) bis (e) zu den unterschiedlichen Zeiten erfüllt ist, wobei die Bedingungen (a) bis (e) einschließen:
(a) eine Bedingung dahingehend, dass durch die Erfassungs-Einheit erfasster Venendruck einen Schwellenwert überschreitet;
(b) eine Bedingung dahingehend, dass der Venendruck eine vorgegebene Zunahmerate überschreitet;
(c) eine Bedingung dahingehend, dass durch die Erfassungs-Einheit erfasstes Herzminutenvolumen über einen Schwellenwert hinaus abnimmt;
(d) eine Bedingung dahingehend, dass das Herzminutenvolumen über eine vorgegebene Abnahmerate hinaus abnimmt; und
(e) eine Bedingung dahingehend, dass ein Abstand zwischen Markierungen zu einer spätesten Zeit und einer Zeit unmittelbar vor der spätesten Zeit ein vorgegebener oder größerer Abstand ist.

16. Vorrichtung (180) für hämodynamische Parameteranalyse nach Anspruch 12;
wobei die Anzeigeeinrichtung (151) ausgeführt ist zum
Umschalten zwischen einem ersten Bildschirm zum Anzeigen physiologischer Informationen des Patienten und/oder eines auf Basis der physiologischen Informationen gemessenen Messwertes und einem zweiten Bildschirm, der zum Anzeigen eines Analyseergebnisses hämodynamischer Parameter des Patienten ausgeführt ist, und
wenn der erste Bildschirm angezeigt wird, Anzeigen, dass eine Möglichkeit von Kongestion besteht, auf dem ersten Bildschirm, in einem Fall, in dem durch die Erfassungs-Einheit erfasster Venendruck den Schwellenwert von Venendruck übersteigt, und Anzeigen, dass eine Möglichkeit von peripherem Kreislaufversagen vorliegt, auf dem ersten Bildschirm in einem Fall, in dem durch die Erfassungs-Einheit erfasstes Herzminutenvolumen den Schwellenwert von Herzminutenvolumen übersteigt.

17. Vorrichtung (180) für hämodynamische Parameteranalyse nach einem der Ansprüche 7, 8, 9 und 11 bis 15,
wobei die Anzeigeeinrichtung (151) so ausgeführt ist, dass sie ein Ergebnis von Analyse hämodynamischer Parameter des Patienten zusammen mit physiologischen Informationen des Patienten und/oder einen auf Basis der physiologischen Informationen gemessenen Messwert anzeigt, wobei die physiologischen Informationen und der Messwert mit dem Ergebnis von Analyse zusammenhängen.

18. Vorrichtung (180) für hämodynamische Parameteranalyse nach Anspruch 17,
wobei die Erfassungs-Einheit (203) so ausgeführt ist,
dass sie Venendruck und Herzminutenvolumen des Patienten zu unterschiedlichen Zeiten erfasst, und
wobei die Anzeigeeinrichtung (151) so ausgeführt ist, dass sie ein Ergebnis von Analyse hämodynamischer Parameter des Patienten zu den unterschiedlichen Zeiten zusammen mit physiologischen Informationen des Patienten und/oder einen auf Basis der physiologischen Informationen gemessenen Messwert anzeigt, wobei die physiologischen Informationen und der Messwert mit jedem Ergebnis von Analyse zusammenhängen.

19. Vorrichtung (180) für hämodynamische Parameteranalyse nach Anspruch 18, die des Weiteren umfasst:
eine Einheit (208) zum Festlegen einer Prioritäten-Reihenfolge, die so ausgeführt ist, dass sie Prioritäten-Reihenfolge zum Anzeigen physiologischer Informationen des Patienten und/oder eines auf Basis der physiologischen Informationen gemessenen Messwertes festlegt,
wobei die Anzeigeeinrichtung (151) so ausgeführt ist, dass sie physiologische Informationen des Patienten und/oder einen auf Basis der physiologischen Informationen gemessenen Messwert entsprechend einer durch die Einheit zum Festlegen einer Prioritäten-Reihenfolge festgelegten Prioritäten-Reihenfolge anzeigt.

20. Vorrichtung (180) für hämodynamische Parameteranalyse nach Anspruch 13;
wobei die Anzeigeeinrichtung (151) so ausgeführt ist, dass sie, wenn zu den unterschiedlichen Zeiten aufgrund durch eine medizinische Fachkraft für den Patienten durchgeführter Behandlung erfasster Venendruck über einen Schwellenwert hinaus abnimmt oder zunimmt oder erfasstes Herzminutenvolumen über einen Schwellenwert hinaus abnimmt oder zunimmt, Informationen anzeigt, die sich auf die durch die medizinische Fachkraft durchgeführte Behandlung sowie eine Zeit beziehen, zu der die Behandlung durchgeführt wird.

21. Programm für hämodynamische Parameteranalyse, mit dem ein Computer veranlasst wird, Verarbeitung auszuführen, die umfasst:
einen ersten Schritt zum Erfassen von Venendruck sowie Herzminutenvolumen, die auf Basis physiologischer Informationen eines Patienten berechnet werden;
einen zweiten Schritt zum Analysieren hämodynamischer Parameter des Patienten auf Basis des Venendrucks sowie des Herzminutenvolumens, die in dem ersten Schritt erfasst werden,
Festlegen eines Schwellenwertes für Venendruck und Herzminutenvolumen,
Schätzen eines Grades an Kongestion des Patienten, der auf Basis des Venendrucks zu schätzen ist, sowie eines Grades an Kreislaufversagen des Patienten, der auf Basis des Herzminutenvolumens zu schätzen ist, und Bestimmen einer Klassifizierung, die dem Grad an Kongestion des Patienten sowie dem Grad an Kreislaufversagen des Patienten entspricht, auf Basis einer Klassifizierungsgruppe, die sich auf Kongestion und Kreislaufversagen bezieht, wobei die Klassifizierungsgruppe auf Basis des Schwellenwertes von Venendruck und des Schwellenwertes von Herzminutenvolumen klassifiziert wird.

22. Programm für hämodynamische Parameteranalyse, mit dem ein Computer veranlasst
wird, Verarbeitung nach Anspruch 21 auszuführen, die umfasst:
in dem ersten Schritt nicht-invasives Berechnen von Venendruck auf Basis eines Signals, das von einem Sensor erfasst wird, der sich in Kontakt mit oder nahe an einer Körperoberfläche eines Patienten befindet, und Erfassen von Herzminutenvolumen, das nicht-invasiv auf Basis einer Pulszahl des Patienten sowie Pulswellenlaufzeit, berechnet wird, die auf Basis einer Pulswelle ermittelt wird; und
in dem zweiten Schritt Anzeigen des Venendrucks sowie des Herzminutenvolumens, die in dem ersten Schritt erfasst werden, auf einer Koordinatenebene mit Venendruck und Herzminutenvolumen als Koordinatenachsen.

## Revendications

1. Appareil d'analyse des paramètres hémodynamiques (180) comprenant :
une unité d'acquisition (203) configurée pour acquérir la pression veineuse et le débit cardiaque, qui sont calculés à partir d'informations physiologiques d'un sujet ;
une unité d'analyse des paramètres hémodynamiques (204) configurée pour analyser les paramètres hémodynamiques du sujet sur la base de la pression veineuse et du débit cardiaque acquis par l'unité d'acquisition (203), et
une unité de définition de valeurs seuils (206) configurée pour définir une valeur seuil pour la pression veineuse et le débit cardiaque
dans lequel l'unité d'analyse des paramètres hémodynamiques (204) est configurée pour estimer un degré de congestion du sujet à évaluer sur la base de la pression veineuse et un degré d'insuffisance circulatoire du sujet à évaluer sur la base du débit cardiaque, et
dans lequel l'unité d'analyse des paramètres hémodynamiques (204) est configurée pour déterminer une classification correspondant au degré de congestion du sujet et au degré d'insuffisance circulatoire du sujet sur la base d'un groupe de classification lié à la congestion et à l'insuffisance circulatoire, ledit groupe de classification étant classé sur la base de la valeur seuil de la pression veineuse et de la valeur seuil du débit cardiaque.

2. Appareil d'analyse des paramètres hémodynamiques (180) selon la revendication 1,
dans lequel l'unité d'analyse des paramètres hémodynamiques (204) est configurée pour analyser au moins l'une parmi la congestion du sujet et l'insuffisance circulatoire du sujet sur la base de la pression veineuse et du débit cardiaque.

3. Appareil d'analyse des paramètres hémodynamiques (180) selon la revendication 1 ou 2,
dans lequel l'unité de définition de valeurs seuils (206) est configurée pour ajuster une valeur seuil de la pression veineuse et/ou une valeur seuil du débit cardiaque en fonction d'au moins un élément parmi une caractéristique du sujet, notamment son sexe et son âge, et l'état pathologique du sujet.

4. Appareil d'analyse des paramètres hémodynamiques (180) selon l'une quelconque des revendications 1 à 3, comprenant en outre :
une unité de notification (207),
dans lequel l'unité d'acquisition (203) est configurée pour acquérir la pression veineuse et le débit cardiaque du sujet à différents moments, et
dans lequel l'unité de notification (207) est configurée pour déclencher une alarme lorsqu'au moins l'une des conditions (a) à (d) est remplie au cours de ces différents moments, les conditions (a) à (d) incluant :
(a) une condition selon laquelle la pression veineuse acquise par l'unité d'acquisition dépasse une valeur seuil ;
(b) une condition selon laquelle la pression veineuse dépasse un taux d'augmentation prédéterminé ;
(c) une condition selon laquelle le débit cardiaque acquis par l'unité d'acquisition diminue au-delà d'une valeur seuil ; et
(d) une condition selon laquelle le débit cardiaque diminue au-delà d'un taux de diminution prédéterminé.

5. Appareil d'analyse des paramètres hémodynamiques (180) selon l'une quelconque des revendications 1 à 4, comprenant en outre :
une unité d'acquisition d'informations physiologiques configurée pour acquérir des informations physiologiques du sujet,
dans lequel l'unité d'acquisition (203) est configurée pour acquérir la pression veineuse et le débit cardiaque du sujet à différents moments, et
dans lequel l'unité d'acquisition d'informations physiologiques (201) est configurée pour augmenter la fréquence d'acquisition des informations physiologiques du sujet lorsqu'au moins l'une des conditions (a) à (d) est remplie au cours de ces différents moments, les conditions (a) à (d) incluant :
(a) une condition selon laquelle la pression veineuse acquise par l'unité d'acquisition dépasse une valeur seuil ;
(b) une condition selon laquelle la pression veineuse dépasse un taux d'augmentation prédéterminé ;
(c) une condition selon laquelle le débit cardiaque acquis par l'unité d'acquisition diminue au-delà d'une valeur seuil ; et
(d) une condition selon laquelle le débit cardiaque diminue au-delà d'un taux de diminution prédéterminé.

6. Appareil d'analyse des paramètres hémodynamiques (180) selon l'une quelconque des revendications 1 à 5, comprenant en outre
une unité de sortie (150) configurée pour fournir un résultat d'analyse des paramètres hémodynamiques du sujet analysé par l'unité d'analyse des paramètres hémodynamiques (204).

7. Appareil d'analyse des paramètres hémodynamiques (180) selon la revendication 6,
dans lequel l'unité de sortie (150) inclut un écran (151) configuré pour afficher le résultat de l'analyse, et
dans lequel l'écran (151) est configuré pour afficher un résultat d'analyse des paramètres hémodynamiques du sujet sur un plan de coordonnées dont les axes sont la pression veineuse et le débit cardiaque.

8. Appareil d'analyse des paramètres hémodynamiques (180) selon la revendication 1,
comprenant en outre un écran (151) configuré pour afficher la pression veineuse et le débit cardiaque, qui sont acquis par l'unité d'acquisition (203), sur un plan de coordonnées dont les axes sont la pression veineuse et le débit cardiaque ;
dans lequel l'unité d'acquisition (203) est configurée pour calculer la pression veineuse de manière non invasive à partir d'un signal acquis par un capteur en contact avec ou à proximité d'une surface corporelle d'un sujet, et pour acquérir le débit cardiaque calculé de manière non invasive à partir de la fréquence cardiaque du sujet et du temps de transit de l'onde de pouls obtenu à partir d'une onde de pouls.

9. Appareil d'analyse des paramètres hémodynamiques (180) selon la revendication 8,
dans lequel l'unité d'acquisition (203) est configurée pour calculer de manière non invasive la pression veineuse à partir d'une onde de pouls acquise auprès du sujet et de la pression appliquée au sujet.

10. Appareil d'analyse des paramètres hémodynamiques (180) selon l'une quelconque des revendications 1 à 9, comprenant en outre
une unité de calcul (202) configurée pour calculer la pression veineuse et le débit cardiaque du sujet à partir d'informations physiologiques du sujet,
dans lequel l'unité d'acquisition (203) est configurée pour acquérir la pression veineuse et le débit cardiaque calculés par l'unité de calcul (202).

11. Appareil d'analyse des paramètres hémodynamiques (180) selon l'une des revendications 7 ou 8,
dans lequel l'écran (151) est configuré pour afficher un degré de congestion et d'insuffisance circulatoire périphérique dans chaque région divisée par la valeur seuil sur le plan de coordonnées.

12. Appareil d'analyse des paramètres hémodynamiques (180) selon la revendication 11,
dans lequel l'unité de définition de valeurs seuils (206) est configurée pour définir respectivement une valeur seuil de pression veineuse et une valeur seuil de débit cardiaque pour la pression veineuse et le débit cardiaque, de manière à diviser le plan de coordonnées en quatre régions, et
dans lequel l'écran (151) est configuré pour
afficher, dans une région où la pression veineuse sur le plan de coordonnées est supérieure à la valeur seuil de la pression veineuse, qu'il existe un risque de congestion, et
afficher, dans une région où le débit cardiaque sur le plan de coordonnées est inférieur à la valeur seuil du débit cardiaque, qu'il existe un risque d'insuffisance circulatoire périphérique.

13. Appareil d'analyse des paramètres hémodynamiques (180) selon l'une quelconque des revendications 7, 8, 9, 11 et 12,
dans lequel l'unité d'acquisition (203) est configurée pour acquérir la pression veineuse et le débit cardiaque du sujet à différents moments, et
dans lequel l'écran (151) est configuré pour afficher l'historique de la pression veineuse et du débit cardiaque à ces différents moments en traçant un marqueur à chaque position du plan de coordonnées correspondant à la pression veineuse et au débit cardiaque.

14. Appareil d'analyse des paramètres hémodynamiques (180) selon la revendication 13, comprenant en outre
une unité de notification (207),
dans lequel l'unité de notification (207) est configurée pour déclencher une alarme lorsqu'au moins l'une des conditions (a) à (e) est remplie au cours des différents moments, les conditions (a) à (e) incluant :
(a) une condition selon laquelle la pression veineuse acquise par l'unité d'acquisition dépasse une valeur seuil ;
(b) une condition selon laquelle la pression veineuse dépasse un taux d'augmentation prédéterminé ;
(c) une condition selon laquelle le débit cardiaque acquis par l'unité d'acquisition diminue au-delà d'une valeur seuil ;
(d) une condition selon laquelle le débit cardiaque diminue au-delà d'un taux de diminution prédéterminé ; et
(e) une condition selon laquelle la distance entre des marqueurs à un instant le plus récent et à un instant immédiatement antérieur à cet instant le plus récent est égale ou supérieure à une distance prédéterminée.

15. Appareil d'analyse des paramètres hémodynamiques (180) selon la revendication 13, comprenant en outre
une unité d'acquisition d'informations physiologiques (201) configurée pour acquérir des informations physiologiques du sujet,
dans lequel l'unité d'acquisition d'informations physiologiques (201) est configurée pour augmenter la fréquence d'acquisition des informations physiologiques du sujet lorsqu'au moins l'une des conditions (a) à (e) est remplie au cours des différents moments, les conditions (a) à (e) incluant :
(a) une condition selon laquelle la pression veineuse acquise par l'unité d'acquisition dépasse une valeur seuil ;
(b) une condition selon laquelle la pression veineuse dépasse un taux d'augmentation prédéterminé ;
(c) une condition selon laquelle le débit cardiaque acquis par l'unité d'acquisition diminue au-delà d'une valeur seuil ;
(d) une condition selon laquelle le débit cardiaque diminue au-delà d'un taux de diminution prédéterminé ; et
(e) une condition selon laquelle la distance entre des marqueurs à un instant le plus récent et à un instant immédiatement antérieur à cet instant le plus récent est égale ou supérieure à une distance prédéterminée.

16. Appareil d'analyse des paramètres hémodynamiques (180) selon la revendication 12, dans lequel l'écran (151) est configuré pour
basculer entre un premier écran configuré pour afficher des informations physiologiques du sujet et/ou une valeur de mesure mesurée sur la base des informations physiologiques, et un deuxième écran configuré pour afficher un résultat d'analyse des paramètres hémodynamiques du sujet, et
afficher, lorsque le premier écran est affiché, qu'il existe un risque de congestion sur le premier écran dans le cas où la pression veineuse acquise par l'unité d'acquisition dépasse la valeur seuil de la pression veineuse, et qu'il existe un risque d'insuffisance circulatoire périphérique sur le premier écran dans le cas où le débit cardiaque acquis par l'unité d'acquisition dépasse la valeur seuil du débit cardiaque.

17. Appareil d'analyse des paramètres hémodynamiques (180) selon l'une quelconque des revendications 7, 8, 9 et 11 à 15,
dans lequel l'écran (151) est configuré pour afficher un résultat d'analyse des paramètres hémodynamiques du sujet ainsi que des informations physiologiques du sujet et/ou une valeur de mesure mesurée sur la base des informations physiologiques, lesdites informations physiologiques et ladite valeur de mesure étant liées au résultat d'analyse.

18. Appareil d'analyse des paramètres hémodynamiques (180) selon la revendication 17,
dans lequel l'unité d'acquisition (203) est configurée pour acquérir la pression veineuse et le débit cardiaque du sujet à différents moments, et
dans lequel l'écran (151) est configuré pour afficher un résultat d'analyse des paramètres hémodynamiques du sujet à ces différents moments, ainsi que des informations physiologiques du sujet et/ou une valeur de mesure mesurée sur la base des informations physiologiques, lesdites informations physiologiques et ladite valeur de mesure étant liées à chaque résultat d'analyse.

19. Appareil d'analyse des paramètres hémodynamiques (180) selon la revendication 18, comprenant en outre
une unité de définition d'ordre de priorité (208) configurée pour définir un ordre de priorité pour l'affichage des informations physiologiques du sujet et/ou d'une valeur mesurée sur la base des informations physiologiques,
dans lequel l'écran (151) est configuré pour afficher les informations physiologiques du sujet et/ou une valeur mesurée sur la base de ces informations physiologiques selon l'ordre de priorité défini par l'unité de définition d'ordre de priorité.

20. Appareil d'analyse des paramètres hémodynamiques (180) selon la revendication 13,
dans lequel l'écran (151) est configuré pour afficher, lorsque la pression veineuse acquise diminue ou augmente au-delà d'une valeur seuil ou lorsque le débit cardiaque acquis diminue ou augmente au-delà d'une valeur seuil à différents moments en raison d'un traitement administré par un professionnel de santé au sujet, des informations relatives au traitement administré par le professionnel de santé et l'instant auquel le traitement est administré.

21. Programme d'analyse des paramètres hémodynamiques destiné à amener un ordinateur à exécuter un traitement, comprenant :
une première étape consistant à acquérir la pression veineuse et le débit cardiaque qui sont calculés sur la base d'informations physiologiques d'un sujet ;
une deuxième étape consistant à analyser les paramètres hémodynamiques du sujet sur la base de la pression veineuse et du débit cardiaque acquis lors de la première étape,
à définir une valeur seuil pour la pression veineuse et le débit cardiaque,
l'estimation d'un degré de congestion du sujet à évaluer sur la base de la pression veineuse et d'un degré d'insuffisance circulatoire du sujet à évaluer sur la base du débit cardiaque, et
la détermination d'une classification correspondant au degré de congestion du sujet et au degré d'insuffisance circulatoire du sujet sur la base d'un groupe de classification lié à la congestion et à l'insuffisance circulatoire, le groupe de classification étant déterminé en fonction de la valeur seuil de la pression veineuse et de la valeur seuil du débit cardiaque.

22. Programme d'analyse des paramètres hémodynamiques destiné à amener un ordinateur à exécuter un traitement selon la revendication 21, comprenant :
dans la première étape, le calcul de manière non invasive de la pression veineuse sur la base d'un signal acquis par un capteur en contact avec ou à proximité d'une surface corporelle d'un sujet, et l'acquisition du débit cardiaque calculé de manière non invasive sur la base de la fréquence cardiaque du sujet et du temps de transit de l'onde de pouls obtenu à partir d'une onde de pouls ; et
dans la deuxième étape, l'affichage de la pression veineuse et du débit cardiaque acquis lors de la première étape sur un plan de coordonnées dont les axes sont respectivement la pression veineuse et le débit cardiaque.
